# FASCICULE DE BREVET EUROPEEN

(11) **EP 3 233 209 B1**
(45) Date de publication et mention de la délivrance du brevet: **27.03.2019**
(21) Numéro de dépôt: 15813342.1
(22) Date de dépôt: 15.12.2015
(51) Int. Cl.: A61Q 15/00, A61K 8/73, C08B 37/08

(54) **PROCÉDÉ COSMÉTIQUE POUR ATTÉNUER LES ODEURS**
KOSMETISCHES VERFAHREN ZUR KONTROLLE VON GERÜCHEN
COSMETIC METHOD FOR CONTROLLING ODORS

(30) Priorité: 16.12.2014 FR 1462491
(43) Date de publication de la demande: 25.10.2017
(73) Titulaire: L'Oréal, 75008 Paris (FR)
(72) Inventeur: GREAVES, Andrew, 77700 Magny-le-Hongre (FR)
(74) Mandataire: Kromer, Christophe
(86) Numéro de dépôt international: PCT/EP2015/079860
(87) Numéro de publication internationale: WO 2016/096897

(56) Documents cités:
- WO-A1-2014/081391
- WO-A2-02/088189
- DE-A1-102013 202 122
- KR-A- 20130 138 563
- US-A1- 2003 049 290
- US-A1- 2004 192 646
- US-A1- 2005 208 309

## Description

La présente invention concerne un procédé de traitement des odeurs, notamment un procédé cosmétique de traitement des corporelles humaines, mettant en oeuvre une composition comprenant un polymère de chitosane greffé d'un groupement photoactif particulier, et l'exposition à la lumière de la peau traitée.

Dans le domaine cosmétique, il est bien connu d'utiliser en application topique, des produits déodorants contenant des substances actives de type anti-transpirant ou de type déodorant pour diminuer voire supprimer les odeurs corporelles, notamment axillaires, qui sont généralement désagréables.

La sueur eccrine ou apocrine est généralement peu odorante lorsqu'elle est sécrétée. C'est sa dégradation par les bactéries via des réactions enzymatiques qui produit des composés malodorants. Les actifs déodorants ont ainsi pour fonction de diminuer ou d'empêcher la formation des mauvaises odeurs.

Les substances déodorantes détruisent en général la flore bactérienne résidente. Parmi ces substances, les plus employées sont le Triclosan (2,4,4'-trichloro-2'-hydroxydiphényléther) et le farnésol qui présentent généralement l'inconvénient de modifier de façon importante l'écologie de la flore cutanée. De plus, le Triclosan présente le désavantage d'être inhibé par la présence de certains composés, comme par exemple les tensioactifs non ioniques, couramment utilisés dans la formulation de compositions cosmétiques. Enfin, le caractère insoluble du Triclosan dans l'eau ne permet pas non plus son incorporation dans des formules essentiellement aqueuses.

Les substances déodorantes peuvent également diminuer la croissance des bactéries. Parmi ces substances, on peut citer les chélatants de métaux de transition comme l'acide éthylène diamine tétraacétique (EDTA) ou l'acide diethylenetriaminepentaacétique (DPTA). Ces matériaux présentent l'inconvénient de priver le milieu des métaux nécessaires à la croissance des bactéries.

Il existe donc encore un réel besoin de développer des composés actifs qui présentent une activité déodorante satisfaisante et qui sont faciles à formuler dans des compositions destinées à diminuer la transpiration et/ou les odeurs, en particulier chez l'être humain, et plus particulièrement pour lutter contre les odeurs corporelles, et plus spécifiquement les odeurs axillaires.

Des polymères de chitosane greffé de groupements photoactifs sont décrits dans les documents suivants :
La publication Kim et al, "Preparation of photo-reactive azidophenyl chitosan derivative for immobilization of growth factors", Journal of Applied Polymer Science, Volume 117 (2010), Issue 5, pages 3029-3037 décrit un chitosane dont les groupes amino sont greffés de groupement 4-azidobenzoyle.
La publication S.R. Jameela et al, "Preparation and evaluation of photo-crosslinkable chitosan as a drug delivery matrix", Journal of Applied Polymer Science, vol 86, 1873-1877 (2002) décrit un chitosane dont les groupes amino sont greffés par un groupement 3-azido 2-hydroxy propyle.

La publication S. Aiba et al, "Covalent immobilization of chitosan derivatives onto polymeric film surfaces with the use of a photosensitive hetero-bifunctional crosslinking reagent". Biomaterials, 1987, 8(6):481-488 décrit un chitosane dont les groupes amino sont greffés par un groupement 4-azidobenzènecarboximidoyle.
La demande DE 10 2013 202122décrit un procédé pour traiter les odeurs corporelles humaines comprenant une étape d'exposition de la peau avec un rayonnement lumineux. La demande US 2003/049290 décrit un procédé de traitement de la transpiration et des odeurs corporelles avec l'application sur la peau d'un polymère filmogène de chitosane. Les demandes US 2004/192646 et KR 2013-0138563 décrivent des chitosanes modifiés par des groupements azide et leur application en cosmétique. La demande WO02/088189 décrit des polysaccharides à groupement photoactif. La demande WO 2014/081391 décrit des polymères greffés par des groupes diazirines. La demande US 2005/208309 décrit un chitosane greffé par des groupements aryl-diazirine.

Les inventeurs ont découvert que l'application topique sur la peau d'un polymère de chitosane ayant des groupes amino greffés de groupements photoactifs de type azide ou diazirine, et combinée à l'exposition de la peau traitée avec un rayonnement lumineux, forme un film présentant un effet déodorant sur la peau. Le film obtenu Cet effet déodorant présente une bonne résistance à l'eau et aux frottements. L'effet déodorant du film est ainsi également rémanent à l'eau.

De façon plus précise, la présente invention a pour objet un procédé cosmétique pour traiter les odeurs corporelles humaines en particulier les odeurs axillaires et éventuellement la transpiration humaine comprenant :
(i) une étape consistant à appliquer sur la peau une composition, notamment cosmétique, comprenant, dans un milieu physiologiquement acceptable, un polymère de chitosane dont les groupes amino sont greffés de groupements photoactif de type azide ou diazirine de formule (I) tel que défini ci-après
(ii) une étape consistant à exposer la peau à un rayonnement lumineux, de préférence pendant au moins 5 seconde; l'étape consistant à appliquer un rayonnement lumineux étant effectuée après ou en même temps que l'étape consistant à appliquer la composition cosmétique comprenant le chitosane greffé (I). Cette étape peut être répétée plusieurs fois pendant la journée.
Le procédé mis en oeuvre selon l'invention permet de masquer, absorber, améliorer et/ou diminuer l'odeur désagréable résultant de la décomposition de la sueur humaine. En outre, l'action déodorante du procédé mis en oeuvre présente une bonne résistance à l'eau et donc l'action est rémanente après le lavage de la peau traitée. De plus, le film obtenu déposé sur la peau présente une bonne résistance aux frottements et présente ainsi une bonne tenue dans le temps.
Le polymère de chitosane greffé mis en oeuvre dans le procédé selon l'invention est un polymère de formule (I) (voir la revendication 1), dans laquelle R' représente indépendamment H ou un groupement acétyle (CH₃C(O)-) ou un groupement -CO-L-X , le polymère renfermant au moins un groupement R' =-CO-L-X,
L étant un groupe divalent hydrocarboné comprenant de 1 à 20 atomes de carbone, de préférence de 2 à 10 atomes de carbone, linéaire, ramifiée ou cyclique, saturé ou insaturé, et pouvant être interrompu par un ou plusieurs hétéroatomes non adjacents choisis parmi l'oxygène, le soufre ou groupements -NH-, -CO-, -CONH-, -COO-, -O-CO-N(Ra)-, en particulier-O-CO-NH-, -N(Rb)-CO-N(Rc)-, en particulier-NH-CO-NH-, ledit groupe divalent pouvant éventuellement être substitué pour un ou plusieurs groupes choisi parmi les groupes hydroxyle, amine, acide carboxylique, amide, cyano, acyl(C₁-C₄)amino,
avec Ra, Rb, Rc désignant indépendamment un atome d'hydrogène ou un radical alkyle C₁-C₆ linéaire ou ramifié , de préférence linéaire ;
X désigne un groupe photoactif de type azide ou diazirine ;
n varie de 5 à 2000.

Ainsi, le chitosane greffé (I) peut également se représenter selon la formule (la) : 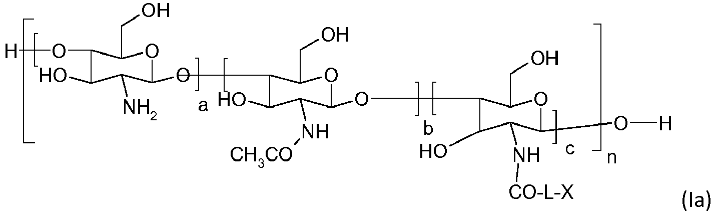
dans laquelle le chitosane greffé comprend a motifs portant un groupe amino libre, b motifs portant un groupe amino N-acétylé et c motifs portant un groupe amino greffé d'un groupe photoactif -CO-L-X ;
b va de 0 à 0,5, de préférence de 0,05 à 0,30, et mieux de 0,1 à 0,30 ;
c va de 0,001 à 0,5 , de préférence de 0,01 à 0,3 , et mieux de 0,01 à 0,2 ;
et a + b + c = 1
X , L et n ayant les significations décrites précédemment.

Par exemple, lorsque a = 0,5 , b = 0,3 et c = 0,2, cela signifie que 50 % des groupes amino sont libres (non substitués), 30 % des groupes amino sont acétylés et 20 % des groupes amino sont greffés par le groupement -CO-L-X, correspondant au polymère de chitosane de formule :

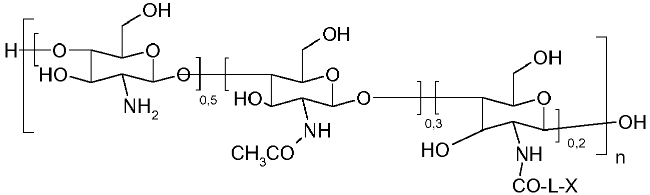

X est en particulier un groupe photoactif tel que le groupe azide (a) ou le groupe diazirine (b) :

| | |
|---|---|
| 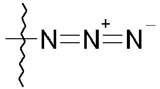 | 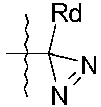 |
| (a) | (b) |

avec Rd désignant H ou un radical alkyle en C₁-C₆ , linéaire ou ramifié, de préférence linéaire, ou un radical CF₃ ;
De préférence, le chitosane greffé a un taux de greffage (taux molaire) en groupements photoactifs (-CO-L-X) allant de 0,1 à 50 %, préférentiellement allant de 1 à 30 %, et plus préférentiellement allant de 1 à 20 %.

De préférence, L est un radical divalent hydrocarboné en C₁-C₂₀, de préférence en C₁-C₁₀, linéaire, ramifié ou cyclique, saturé ou insaturé (dont aromatique), pouvant être interrompu par un ou plusieurs hétéroatomes non adjacents choisi parmi l'oxygène ou par un ou plusieurs groupements non adjacents choisis parmi -NH-, -CO-, -O-CO-, -NH-CO- ;
De préférence, X représente un groupement photoactif choisi parmi :

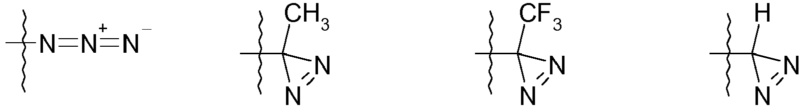

Avantageusement, le chitosane greffé (I) a un degré d'acétylation (-CO-CH₃) allant de 0 à 50 %, de préférence allant de 5 à 30 %, et plus préférentiellement allant de 10 à 30 %.

Avantageusement, n varie de 5 à 1700, de préférence de 5 à 280, plus préférentiellement de 15 à 165.

De préférence, L désigne un radical divalent choisi parmi :

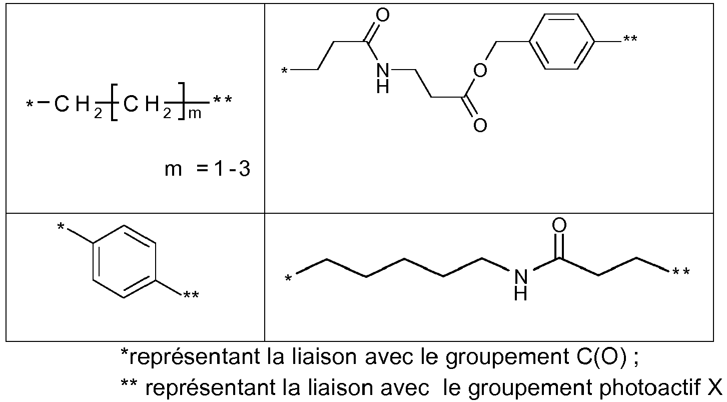

De préférence, le groupement -CO-L-X est choisi parmi :

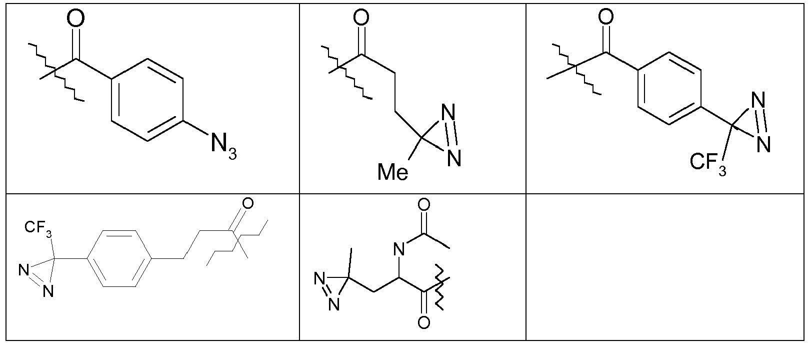

et préférentiellement choisi parmi :

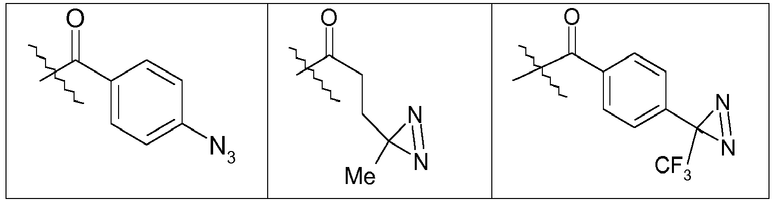

L'invention a également pour objet les polymères greffés nouveaux de formule (II) 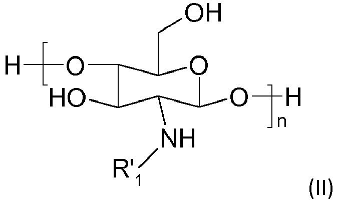 dans laquelle R'₁ représente indépendamment H ou un groupement acétyle ou un groupement -CO-L-X de formule (c) suivante : 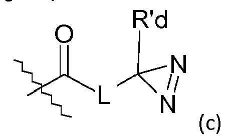 dans laquelle :
L a la signification décrite précédemment ;
R'd désigne H ou un radical alkyle en C₁-C₄ linéaire ou ramifié, de préférence linéaire, ou un radical CF₃ ;
, le polymère renfermant au moins un groupement- CO-L-X de formule (c)
n varie de 5 à 1700, de préférence de 5 à 280, plus préférentiellement de 15 à 165.

De préférence, L est un radical divalent hydrocarboné en C₁-C₂₀, linéaire, ramifié ou cyclique, saturé ou insaturé (dont aromatique), pouvant être interrompu par un ou plusieurs hétéroatomes non adjacents choisi parmi l'oxygène ou par un ou plusieurs groupements non adjacents choisis parmi -NH-, -CO-, -O-CO- , -NH-CO-.

De préférence R'd est choisi parmi un atome d'hydrogène, un radical méthyle, un radical CF₃.
De préférence, le chitosane greffé (II) a un taux (taux molaire) de greffage en groupements photoactifs -CO-L-X allant de 0,1 à 40 %, préférentiellement allant de 1 à 45 %, et plus préférentiellement allant de 1 à 35 %.
c va de 0,001 à 0,4 , de préférence de 0,01 à 0,35 , et mieux de 0,01 à 0,3 ;
avec a + b + c = 1

Avantageusement, le chitosane greffé (II) a un degré d'acétylation allant de 0 à 50 %, de préférence allant de 5 à 45 %, et plus préférentiellement allant de 10 à 35 %.

Avantageusement, n varie de 5 à 1700, de préférence de 5 à 280, plus préférentiellement de 15 à 165.

Le chitosane greffé (II) peut être un chitosane de formule (II') : 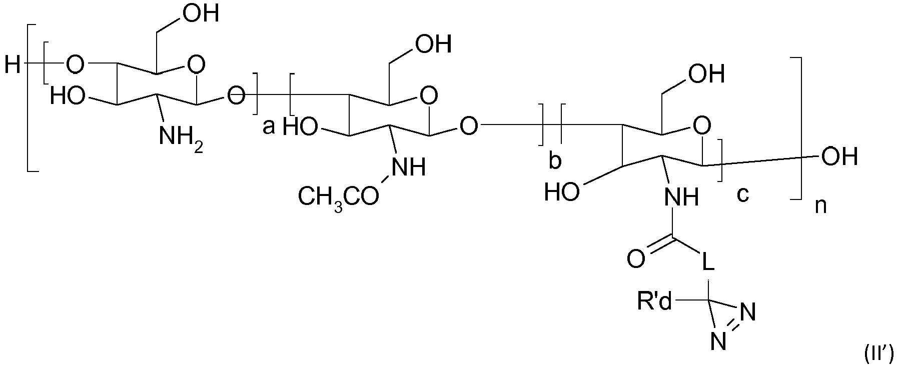 dans laquelle a, b, c , L, R'd ont les même significations que celles décrites précédemment pour les composés (Ib).

Selon un premier mode de réalisation préféré de l'invention, le polymère de chitosane greffé (II) peut être de formule (IIa) suivante : 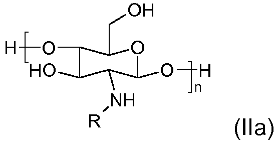 dans laquelle R désigne H ou acétyle ou un groupement (J) : 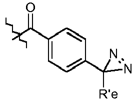 dans lequel R'e = CH₃ ou CF₃

le polymère comprenant au moins un groupement (J). De préférence, le groupement J est le groupement :

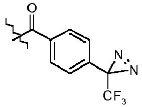

Selon un deuxième mode de réalisation préféré de l'invention, le polymère de chitosane greffé (II) peut être de formule (IIb) suivante : 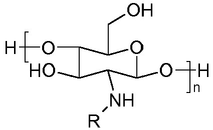 dans laquelle R désigne H ou acétyle ou un groupement (K) : 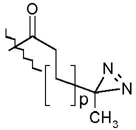 avec p = 1 à 3

le polymère comprenant au moins un groupement (K). De préférence, le groupement K est le groupement :

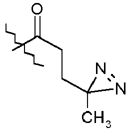

Les composés (II) peuvent être obtenus selon un premier mode de préparation par la réaction d'un chitosane avec un acide carboxylique (A) X-L-COOH à l'aide d'un réactif de couplage (B) pour former un acide activé (C) et permettre la formation d'une liaison amide. Le réactif de couplage est connu de l'homme du métier : on peut utiliser par exemple les carbodimides tel que l'hydrochlorure de 1-éthyl-3-(3-diméthylaminopropyl)carbodiimide (EDC), les alcoxytriazines tel que le chlorure de 4-(4,6-dimethoxy-1,3,5-triazin-2-yl)-4-methylmorpholinium et les -(hydroxyimino)acétates.

La réaction peut être effectuée en présence d'un deuxième activateur d'acide carboxylique par exemple les N-hydroxybenzotriazoles tel que le 1-hydroxybenzotriazole et les N-hydroxysuccinimides tel que le N-hydroxysulfosuccinimide.

La réaction peut se faire dans un solvant aprotique ou protique. De préférence la réaction est faite dans l'eau à un pH compris entre 4 et 9 et préférablement entre 5 et 7. La réaction peut être effectuée à une température comprise entre 5 et 80°C. De préférence la réaction est faite à température ambiante (25 °C).

Une telle réaction de couplage est notamment décrite dans les articles suivants : Catalytic amide formation from non-activated carboxylic acids and amines Chem. Soc. Rev., 2014,43, 2714-2742; Evolution of amide bond formation ARKIVOC 2010 (viii) 189-250 ; Amide bond formation: beyond the myth of coupling reagents Chem. Soc. Rev., 2009,38, 606-631.

A la place de l'acide carboxylique (A), on peut utiliser une acide carboxylique activé (B) X-L-CO-O-W dans lequel W représente un groupement activateur tel qu'un groupe issu du N-hydroxysulfosuccinimide ou du 1-hydroxybenzotriazole.

La réaction de synthèse par l'activation de l'acide carboxylique (A) avec l'hydrochlorure de 1-éthyl-3-(3-diméthylaminopropyl)carbodiimide (B) puis greffage sur le chitosane est schématisée dans le schéma 1 ci-dessous (avec formule du chitosane simplifiée).

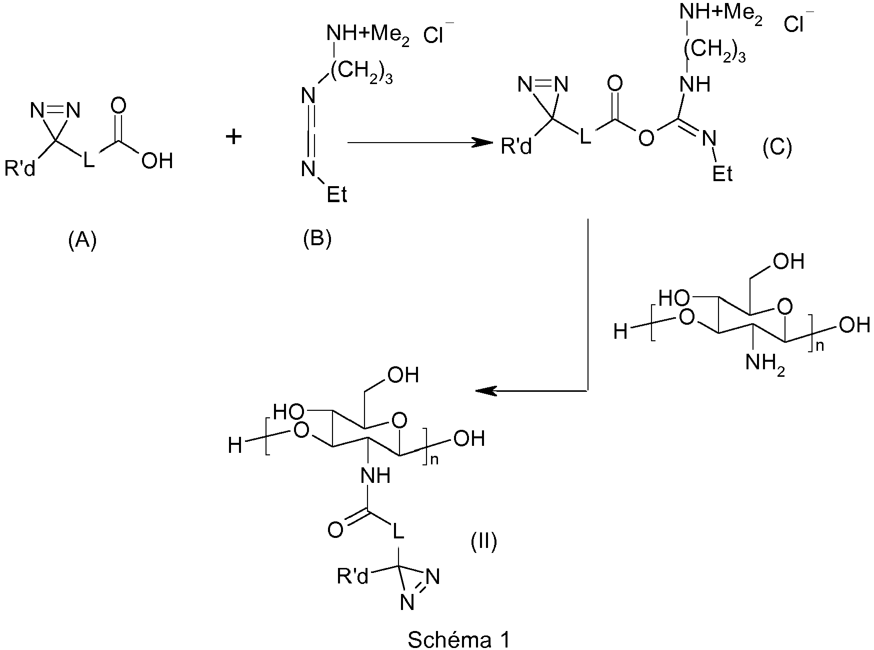

Comme indiqué précédemment, un deuxième activateur tel que le N-hydroxysuccinimide (D) peut être utilisé selon un schéma de synthèse décrit dans le schéma 2 ci-après (avec formule du chitosane simplifiée) :

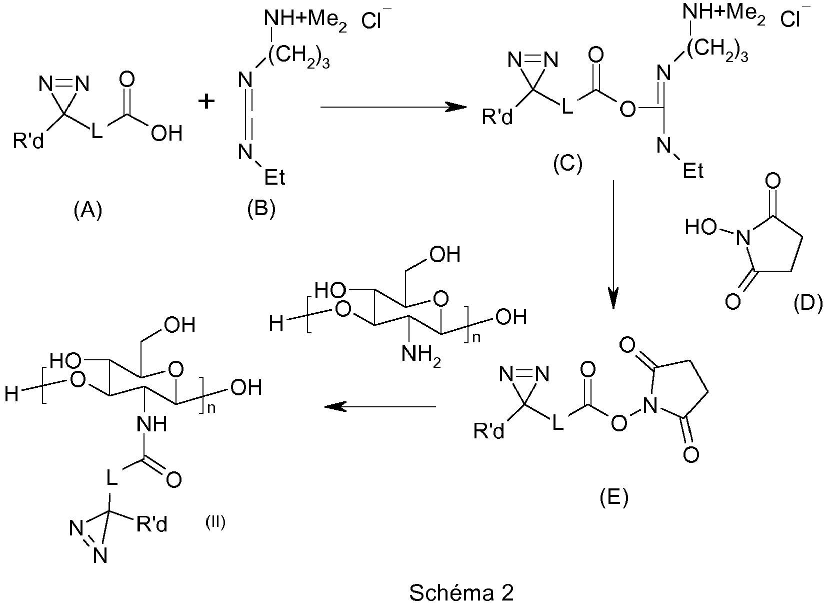

Certains composés (E) sont disponibles tels que ceux décrits dans le tableau ci-après. Ces composés évitent l'utilisation de l'agent de couplage tel que les carbodiimides. Le couplage entre le chitosane et le composé (D) peut être fait directement dans un solvant aprotique ou protique. De préférence la réaction est faite dans l'eau à un pH entre 4 et 9 et préférablement entre 5 et 7. La réaction est faite à température entre 5 et 80°C. De préférence la réaction est faite à température ambiante (25 °C).

| | |
|---|---|
| Ester de l'acide 4-[3-(trifluoromethyl)diazirin-3-yl]benzoïque et du N-hydroxysuccinimide (de la société Toronto Research Chemicals) | 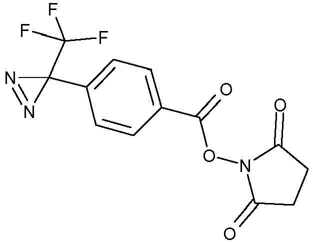 |
| Succinimidyl-diazirine (SDA de la société ThermoScientific) | 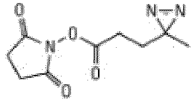 |
| Sulfo-Succinimidyl-diazirine (Sulfo-SDA de la société ThermoScientific) | 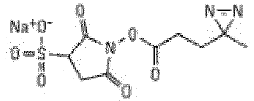 |
| 6-[4,4-azipentanamido]hexanoate de succinimidyle (LC-SDA de la société ThermoScientific) | 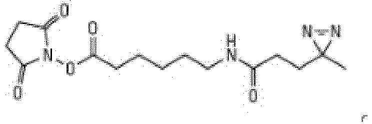 |
| 6-[4,4-azipentanamido]hexanoate de sulfosuccinimidyle (Sulfo-LC-SDA de la société ThermoScientific) | 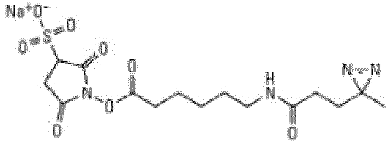 |

Certaines des acides carboxyliques (A) à groupe photoréactif et non-activés sont disponibles dans le commerce tel que ceux cités dans le tableau ci-dessous :

| | |
|---|---|
| acide 4-[3-(trifluoromethyl)-3h-diazirin-3-yl]benzoïque de la société TCI | 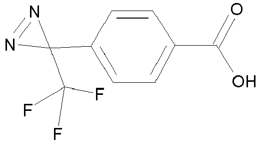 |
| acide alpha-(acetylamino)-3-methyl-3h-diazirine-3-propanoïque de la société ChemStep | 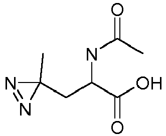 |
| Acide 4-(3-methyl-3h-diazirin-3-yl)butanoïque de la société FineChemie & Pharma | 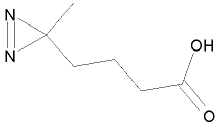 |
| Acide 4-[3-(trifluoromethyl)-3h-diazirin-3-yl]-benzenepropanoïque de la société Dalton Pharma | 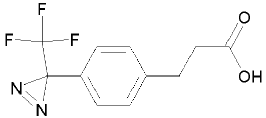 |

L'invention a aussi pour objet une composition comprenant dans un milieu physiologiquement acceptable un polysaccharide greffé (II) ou (IIa) ou (IIb) tel que défini précédemment.

La composition utilisée selon l'invention est généralement adaptée à une application topique sur la peau et comprend donc généralement un milieu physiologiquement acceptable, c'est-à-dire un milieu compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.
Le chitosane greffé (I) ou (Ia) ou (II) ou (IIa) ou (IIb) peut être présent dans la composition utilisée selon l'invention en une teneur allant de 0,1 à 10 % en poids, par rapport au poids total de la composition, de préférence allant de 0,5 % à 10 % en poids, et préférentiellement allant de 1 à 8 % en poids, et plus préférentiellement allant de 1 % à 6 % en poids.

La composition selon l'invention peut se présenter sous toutes les formes galéniques classiquement utilisées pour une application topique et notamment sous forme de dispersions du type lotion ou gel aqueux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion H/E ou d'un gel aqueux.

Avantageusement, la composition utilisée selon l'invention comprend de l'eau, notamment en une teneur pouvant aller de 10 à 99 % en poids, par rapport au poids total de la composition, et de préférence allant de 50 à 99 % en poids.

La composition utilisée selon l'invention peut contenir en outre contenir un ou plusieurs adjuvants couramment utilisés dans le domaine cosmétique, tels que des émulsionnants, des conservateurs, des séquestrants, des parfums, des épaississants, des huiles, des cires, des polymères filmogènes.

Bien entendu, l'homme du métier veillera à choisir ce ou ces éventuels composés additionnels et/ou leur quantité de manière telle que les propriétés anti-rides de la composition selon l'invention ne soient pas, ou substantiellement pas, altérées par l'adjonction envisagée.

Avantageusement, pour le procédé selon l'invention, il est possible d'appliquer 0,01 à 0,5 g de composition cosmétique comprenant le polymère de chitosane greffé, notamment 0,05 à 0,1 g de composition, par cm² de peau.

Le procédé selon l'invention comprend également une étape consistant à exposer la peau à un rayonnement lumineux présentant de préférence une longueur d'ondes comprise entre 360 et 600 nm.

Préférentiellement, dans un premier temps, on applique sur la peau la composition comprenant le polymère de chitosane greffé, puis dans un second temps, on applique un rayonnement lumineux sur la peau.
Il est possible de procéder à une étape de rinçage, par exemple à l'eau, de la peau entre chaque étape du procédé.
De préférence, le rayonnement lumineux utilisé dans le procédé selon l'invention présente une longueur d'ondes comprise entre 400 et 480 nm.
Le rayonnement lumineux présente de préférence une fluence (quantité d'énergie par unité de surface) allant de 3 à 100 J/cm², de préférence allant de 3 à 10 J/cm².
Le rayonnement lumineux peut être une lumière continue ou non continue.
Le rayonnement lumineux peut être la lumière naturelle (lumière du jour).
Le rayonnement lumineux peut être généré par un dispositif, tel que les lampes d'arc telles que les lampes à xénon et les lampes à mercure; les lampes à fluorescence; les lampes à incandescence telles que les halogènes; les LEDs et les lasers.
On peut notamment citer les goLITE BLU de la société Philips, la lampe l'Energylight HF 3319/01 de la société Philips, les lampes Dayvia White et Messa de la société Solvital, la lampe Lumino Plus de la société Lanaform, la lampe medibeam de la société Medibeam, la lampe M-LED 01 de la société Meimed, la lampe Lifemax light pod de la société Lifemax, la lampe Lite-Pad de la société Reicorp, et la lampe Camag Box 3 (4x8W) de la société Camag.
La durée d'exposition au rayonnement lumineux de la peau traitée et apporté par un dispositif est de préférence d'au moins 5 secondes, De préférence cette durée d'exposition peut aller de 10 secondes à 15 minutes, notamment entre 15 secondes et 10 minutes, encore mieux entre 20 secondes et 5 minutes, quel que soit l'ordre des étapes (l'une avant l'autre ou simultanées).
A titre d'exemple, en cas d'application simultanée du rayonnement lumineux apporté par un dispositif et de la composition comprenant le polymère de chitosane greffé, la durée d'exposition à la lumière peut avantageusement aller de 5 secondes à 15 minutes. Il est possible de procéder à un rinçage de la composition.

A titre d'exemple, en cas d'application de la composition selon l'invention, puis d'exposition au rayonnement lumineux apporté par un dispositif, la durée d'exposition à la lumière peut avantageusement être comprise entre 5 secondes et 15 minutes. Il est possible de laisser poser la composition utilisée selon l'invention pendant une durée de 1 seconde à 3 heures, avant de procéder à l'étape d'application du rayonnement lumineux. Il est possible de procéder à un rinçage de la composition, après l'étape d'exposition au rayonnement lumineux.

La durée d'exposition de la peau traitée à la lumière du jour comme rayonnement lumineux est de préférence d'au moins 30 secondes, De préférence cette durée d'exposition peut aller de 30 secondes à 1 heure, notamment entre 30 secondes et 30 minutes, encore mieux entre 1 minute et 15 minutes, quel que soit l'ordre des étapes (l'une avant l'autre ou simultanées).

A titre d'exemple, en cas d'application simultanée de la lumière du jour et de la composition comprenant le chitosane greffé, la durée d'exposition à la lumière peut avantageusement aller de 3 minutes à 12 heures. Il est possible de procéder à un rinçage de la composition.

A titre d'exemple, en cas d'application de la composition comprenant le chitosane greffé, puis d'exposition à la lumière du jour, la durée d'exposition à la lumière peut avantageusement être comprise entre 3 minutes à 12 heures. Il est possible de laisser poser la composition selon l'invention pendant une durée de 1 seconde à 3 heures, avant de procéder à l'étape d'exposition au rayonnement lumineux.
Il est possible de procéder à un rinçage de la composition, après l'étape d'exposition au rayonnement lumineux, mais cela n'est pas obligatoire.

L'étape d'exposition au rayonnement lumineux peut être répétée plusieurs fois pendant la journée.

L'application de la composition cosmétique utilisée selon l'invention se fait selon les techniques habituelles, par exemple par application (notamment de crèmes, de gels, de sérums, de lotions) sur la peau destinée à être traitée, en particulier la peau des aisselles ou des pieds.

Le procédé appliqué sur la peau peut également être appliqué sur des matériaux destinés à désodoriser des environnements malodorants.
Aussi l'invention a également pour objet un procédé pour désodoriser les pieds et/ou les chaussures consistant à disposer dans une chaussure une semelle dont la surface a été traitée par application sur la dite surface d'une composition comprenant un chitosane greffé (I) tel que décrit précédemment puis l'exposition de la surface traitée à un rayonnement lumineux, de préférence pendant au moins 5 secondes.

La semelle peut être en liège, en matière caoutchouc, en cuir.
L'invention a également pour objet un procédé pour désodoriser le corps (ou partie du corps) consistant à habiller le corps (ou partie du corps) avec des vêtements en tissu textile dont la surface a été traitée par application sur la surface du textile d'une composition comprenant un chitosane greffé (I) tel que décrit précédemment puis d'exposer la surface traitée à un rayonnement lumineux, de préférence pendant au moins 5 secondes.
La partie du corps peut être les pieds et le vêtement textile peut être des chaussettes, des bas, un collant. La partie du corps peut être le buste et le vêtement textile un tee-shirt.
L'invention a également pour objet un procédé pour désodoriser les pieds consistant à habiller les pieds avec un vêtement pour les pieds en tissu textile, notamment en forme de chaussettes ou de bas ou de collant, traité par application sur ledit tissu textile d'une composition comprenant un chitosane greffé (I) tel que décrit précédemment puis d'exposer le tissu textile traité à un rayonnement lumineux, de préférence pendant au moins 5 secondes.
Le textile du vêtement peut être du coton, de la laine, de la soie, du lin, du polyamide , du polyester, du poly acide lactique, des chlorofibres, du polyacrylonitrile, de l'élasthane, de l'aramide, du polybenzimidazole, du polypropylène, du polyéthylène, du polyphénol, du polyurée, du polyuréthane, or leurs mélanges. Le traitement est de préférence appliqué sur le textile avant la confection du vêtement avec ledit textile traité.
Le traitement de semelles ou de textile peut être effectué avec une composition aqueuse contenant le polymère de chitosane greffé.
Les conditions d'exposition au rayonnement décrites précédemment s'appliquent à ces différents procédés.

L'invention va maintenant être décrite en référence aux exemples suivants. Les teneurs sont exprimées en pourcentage pondéral.

### Exemple de synthèse 1 (polymère 1): Chitosane fonctionnalisé à 18 % de groupe diazirine

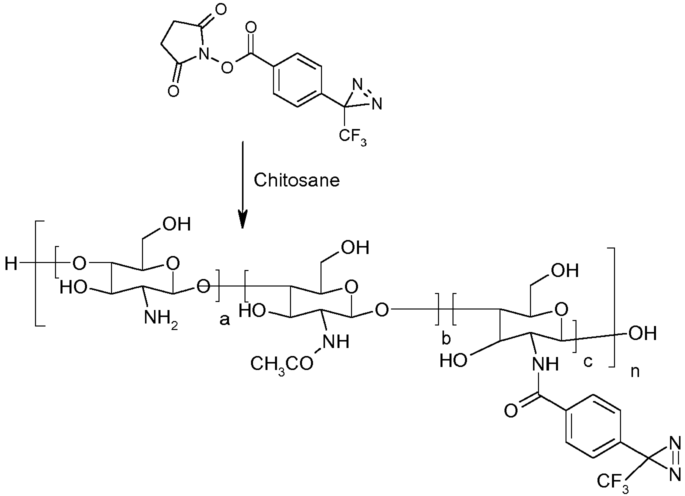

avec a = 0,52 ; b = 0,30 ; c = 0,18
n étant tel que le chitosane non greffé à un poids moléculaire Mn = 28 000 Daltons

Dans un ballon protégé de la lumière 5 g de chitosane (Zenvivo® Protect de chez Clariant avec un degré d'acétylation (%mol) = 30 % et un poids moléculaire Mn = 28 kDa) ont été solubilisé dans 250 g d'une solution aqueuse d'acide acétique à 1 % en poids, Ensuite on a ajouté une solution de 9 g d'ester d'acide 4-[3-(trifluoromethyl)diazirin-3-yl]benzoïque et de N-hydroxysuccinimide dans 81 ml de dioxane. Après 10 minutes d'agitation, le pH a été ajusté à 5 avec une solution aqueuse de bicarbonate de sodium (2M). La solution a été agitée à température ambiante (25 °C) pendant 4 jours. Le mélange réactionnel a ensuite été introduit dans un tube de dialyse (Spectra/Por Dialysis Membrane MWCO 3500; NFW 54mm, Diam. 34mm, Vol/length 9.3ml/cm; Spectrumlabs.com ref 132725) et dialysé dans 2 litres d'eau pendant 4 jours, en remplaçant l'eau 2 fois par jour. La solution aqueuse a été lyophilisée pour donner un produit solide fibreux de couleur jaune. Ce solide récupéré a été lavé à la température ambiante dans un flacon brun à l'aide d'acétone (1 fois avec 1 litre puis 2 fois avec 500 ml).

Le résidu solide a ensuite été filtré pendant 5 minutes puis séché sous vide à la température ambiante pendant 12 heures. On a ainsi obtenu 5,8 g d'un produit solide (poudre) de couleur beige.
Le produit a été conservé dans un flacon de couleur ambre à - 20 °C.
L'analyse RMN 1H dans l'eau deutériée : 18 % de greffage

### Exemple de synthèse 2 (polymère 2): Chitosane fonctionnalisé à 1 % de groupe diazirine

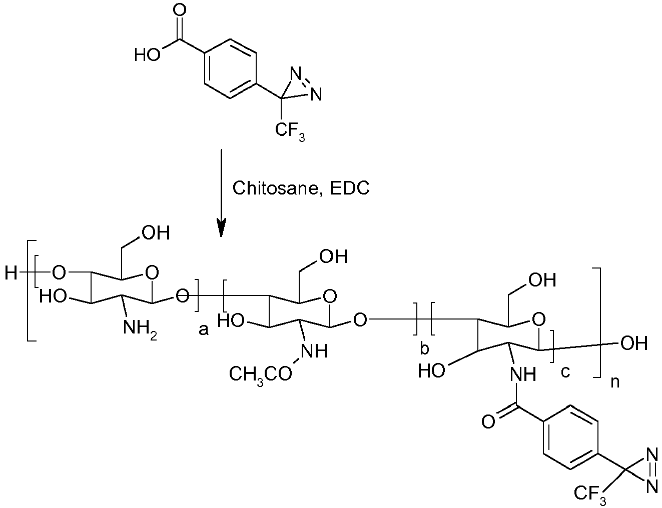

avec a = 0,69 ; b = 0,30 ; c = 0,01
n étant tel que le chitosane non greffé à un poids moléculaire Mn = 28 000 Daltons
0,5 g de chitosane (Zenvivo® Protect de chez Clariant avec un degré d'acétylation = 30% et un poids moléculaire Mn = 28kDa) a été solubilisé dans 25 ml d'une solution aqueuse d'acide acétique à 1 % en poids dans un ballon protégé de la lumière. La solution a été agitée puis un mélange de 10 ml d'éthanol, de 0,38 g d'acide de 4-[3-(trifluoromethyl)diazirin-3-yl]benzoïque et de 0,54 g d'hydrochlorure de 1-éthyl-3-(3-diméthylaminopropyl)carbodiimide (EDC) a été ajouté. Le mélange réactionnel a été agité à 40°C pendant 3 heures, refroidi puis introduit dans un tube de dialyse (Spectra/Por Dialysis Membrane MWCO 3500; NFW 54mm, Diam. 34mm, Vol/length 9.3ml/cm; Spectrumlabs.com ref 132725) et dialysé dans 5 litres d'eau pendant 72 heures, en remplaçant l'eau 6 fois durant cette opération de dialyse. Ensuite la solution a été lyophilisée pour obtenir un produit solide fibreux de couleur jaune pale. Ce solide récupéré a été lavé à la température ambiante à l'aide d'acétone pendant 2 heures (100 ml par lavage en effectuant 3 lavages). Le produit greffé reste insoluble dans l'acétone.
Le résidu solide a ensuite été filtré pendant quelques minutes puis séché sous vide à la température ambiante pendant 12 heures. On a ainsi obtenu 385 mg d'un produit solide (poudre) de couleur beige.
Le produit a été conservé dans un flacon de couleur ambre à - 20 °C.
L'analyse RMN 1H dans l'eau deutériée : 1 % de greffage

### Exemple de synthèse 3 (polymère 3): Chitosane fonctionnalisé à 5 % de groupe diazirine

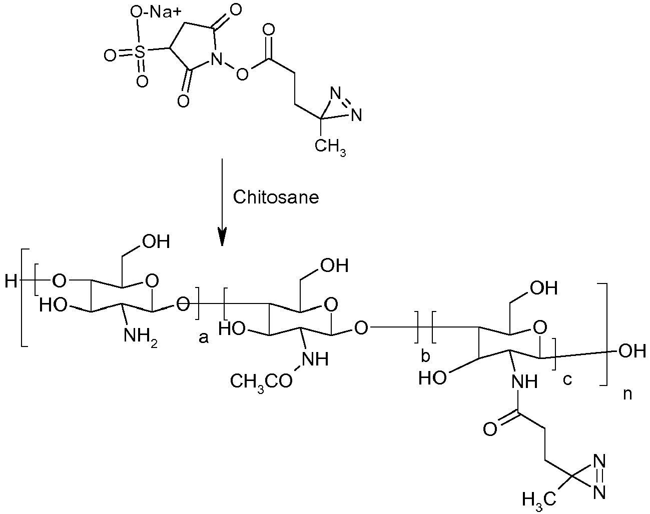

avec a = 0,65 ; b = 0,30 ; c = 0,05
n étant tel que le chitosane non greffé à un poids moléculaire Mn = 28 000 Daltons
200 mg de chitosane (Zenvivo® Protect de chez Clariant avec un degré d'acétylation (%mol) = 30% et un poids moléculaire Mn = 28kDa) a été dissous dans 4,2 ml d'eau distillée dans un ballon recouvert d'une feuille d'aluminium pour empêcher l'exposition à la lumière. 50 mg de succinimidyl 4,4'-azipentanoate (sulfo-SDA de la société ThermoScientific) a été ajouté, sous agitation à température (5 °C). Le mélange réactionnel a été agité pendant 24 h en laissant la température augmenter à 22°C et en gardant le pH entre 4 et 5 par addition d'une solution aqueuse d'acide acétique (1 % en poids). Le mélange réactionnel a ensuite été introduit dans un tube de dialyse (MWCO 3kDa) et dialysé dans 5 litres d'eau osmosée pendant 48 heures, en remplaçant l'eau 6 fois durant cette opération de dialyse. La solution a été lyophilisé pour obtenir un produit solide fibreux de couleur jaune (187mg).
Le produit a été conservé dans un flacon de couleur ambre à - 20 °C .
L'analyse RMN 1H dans l'eau deutériée : 5 % de greffage

### Exemple de synthèse 4 (polymère 4): Chitosane fonctionnalisé à 9 % de groupe azide

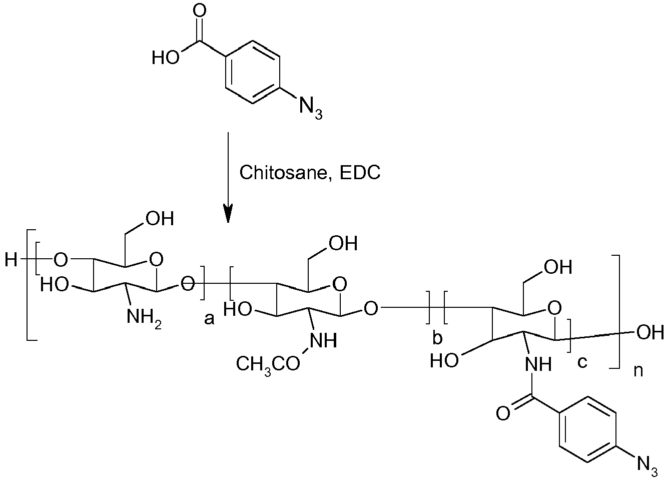

avec a = 0,73 ; b = 0,18 ; c = 0,09
n étant tel que le chitosane non greffé à un poids moléculaire Mn = 28 000 Daltons
0,5 g de chitosane (Zenvivo® Protect de chez Clariant avec un degré d'acétylation (%mol) = 30% et un poids moléculaire Mn = 28kDa) a été solubilisé dans 25 ml d'une solution aqueuse d'acide acétique à 1 % en poids dans un ballon protégé de la lumière. La solution a été agitée puis un mélange de 5 ml d'éthanol, de 0,38 g d'acide 4-azidobenzoique et de 0,54 g d'hydrochlorure de 1-éthyl-3-(3-diméthylaminopropyl)carbodiimide (EDC) a été ajouté. Le mélange réactionnel a été agité à 40°C pendant 3 heures, refroidi puis introduit dans un tube de dialyse (MWCO 3kDa) et dialysé dans 5 litres d'eau pendant 48 heures, en remplaçant l'eau 4 fois durant cette opération de dialyse. Ensuite la solution a été lyophilisée pour obtenir un produit solide fibreux de couleur jaune pale. Ce solide récupéré a été lavé à la température ambiante à l'aide d'acétone pendant 2 heures (100 ml par lavage en effectuant 3 lavages). Le produit greffé reste insoluble dans l'acétone.
Le résidu solide a ensuite été filtré pendant quelques minutes puis séché sous vide à la température ambiante pendant 12 heures. On a ainsi obtenu 360 mg d'un produit solide (poudre) de couleur beige.
Le produit a été conservé dans un flacon de couleur ambre à - 20 °C.
L'analyse RMN 1H dans l'eau deutériée : 9 % de greffage

### Exemple de synthèse 5 (polymère 5): Chitosane fonctionnalisé à 4 % de groupe azide

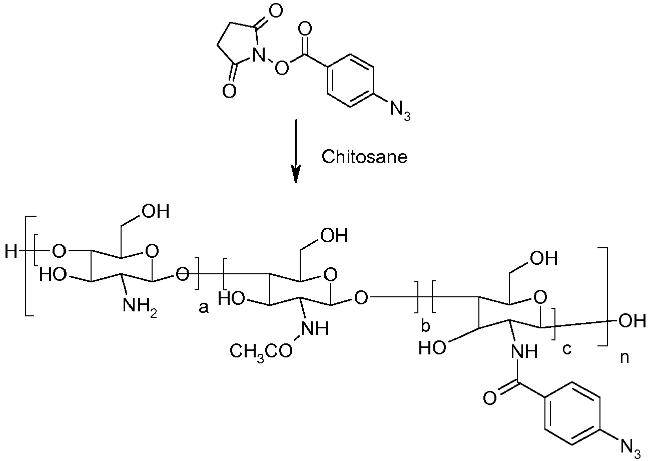

avec a = 0,66 ; b = 0,3 ; c = 0,04
n étant tel que le chitosane non greffé à un poids moléculaire Mn = 28 000 Daltons

Dans un ballon protégé de la lumière 250 mg de chitosane (Zenvivo® Protect de chez Clariant avec un degré d'acétylation (%mol) = 30% et un poids moléculaire Mn = 28kDa) a été solubilisé dans 25 ml d'une solution aqueuse d'acide acétique à 1 % en poids.
Ensuite on a ajouté une solution de 50 mg de 1-{[(4-azidophenyl)carbonyl]oxy}pyrrolidine-2,5-dione dans 3 ml dioxane. Après 10 minutes d'agitation, le pH a été ajusté à 5 avec une solution aqueuse d' hydroxyde de sodium (1M). Le mélange réactionnel a été agité à température ambiante pendant 18 heures. Le mélange réactionnel a ensuite été filtré puis le filtrat a été introduit dans un tube de dialyse ((Spectra/Por Dialysis Membrane MWCO 3500; NFW 54mm, Diam. 34mm, Vol/length 9.3ml/cm; Spectrumlabs.com ref 132725) et dialysé dans 2 litres d'eau pendant 3 heures, en remplaçant l'eau 3 fois. La solution a été lyophilisée pour donner un produit solide fibreux de couleur jaune. Ce solide récupéré a été lavé à la température ambiante dans un flacon brun à l'aide d'acétone (1 fois avec 1 litre puis 2 fois avec 500 ml). Le produit greffé reste insoluble dans l'acétone.
Le résidu solide a ensuite été filtré pendant 5 minutes puis séché sous vide à la température ambiante pendant 12 heures. On a ainsi obtenu 120 g d'un produit solide (poudre) de couleur beige.
Le produit a été conservé dans un flacon de couleur ambre à - 20 °C.
L'analyse RMN 1H dans l'eau deutériée : 4 % de greffage

### Exemples 1 à 8 :

### Mise en évidence de l'effet déodorant des polymères utilisés selon l'invention

On a préparé les compositions suivantes :
Composition 1 : eau
Composition 2 : solution aqueuse à 5 % en poids MA de chitosan Zenzivo Protect de chez Clariant
Composition 3 : solution aqueuse à 5 % en poids MA de polymère 1
Composition 4 : solution aqueuse à 5 % en poids MA de polymère 2
Composition 5 : solution aqueuse à 5 % en poids MA de polymère 3
Composition 6 : solution aqueuse à 5 % en poids MA de polymère 4
Composition 7 : solution aqueuse à 5 % en poids MA de polymère 5

### Préparation du substrat.

Les pièces de stratums cornés humains sont collées sur du scotch (référence 1526886 de la société Office Depot) en assurant que la surface extérieure du stratum cornéum n'est pas en contact directement sur le scotch. Les substrats sont ensuite découpés en bande de taille environ 2 cm longueur et 1cm largeur et utilisés avec le scotch en partie inférieure et la surface extérieure du stratum corné en partie supérieure.
La surface extérieure du stratum cornéum a été nettoyée avec une lingette imprégnée d'éthanol.

### Application des compositions :

### Exemples 1 à 3 :

On a appliqué 100 µl de chacune des compositions 1 à 3 respectivement sur la surface du stratum corneum et laissé pausé 10 minutes puis on a rincé avec 13 ml d'une solution aqueuse 0,9 M de NaCl puis on a essuyé la surface avec un papier absorbant.

### Exemple 4 :

On a appliqué 100 µl de la composition 3 sur la surface du stratum corneum et laissé pausé 5 minutes puis on irradié la surface avec un appareil simulateur de soleil ORIEL de la société ORIEL-LOT pendant 5 minutes. Puis on a rincé avec 13 ml d'une solution aqueuse 0,9 M de NaCl puis on a essuyé la surface avec un papier absorbant.

Les bandes de stratum corneum traitées ont été découpées en petites pièces de taille environ 3 mm x 3 mm et placée dans un flacon en plastique. On a ajouté 100 µl de sueur humaine, fermé et secoué le flacon. Le flacon a ensuite été placé dans un incubateur pendant 24 heures (37 °C, 5 % CO₂).

On a ensuite évalué avec un panel de 4 personnes l'odeur de sueur ressenti en ouvrant chaque flacon (exemples 1 à 4).

On a ensuite mesuré la résistance à l'eau des échantillons de stratum cornéum traités en effectuant un cycle de lavage/rinçage en prenant les pièces de stratum corneum avec une pince, en les trempant pendant 5 secondes dans 10 ml de solution aqueuse à 15 % en poids de lauryl éther sulfate de sodium puis en les rinçant en les trempant pendant 10 secondes dans 20 ml d'eau distillée, et en répétant ce cycle de lavage/rinçage 4 fois. Les pièces de stratum corneum ainsi lavées ont été placées dans un flacon en plastique, on a ajouté 100 µl de sueur humaine. Le flacon fermé a été placé dans un incubateur pendant 24 heures (37 °C, 5 % CO₂).

On a ensuite évalué avec un panel de 5 personnes l'odeur de sueur ressenti en ouvrant chaque flacon (exemples 1a à 4a).

On a recommencé un autre cycle de lavage/rinçage et évalué l'odeur de sueur ressenti (exemples 1b à 4 b).

On a obtenu les résultats suivants :
On a obtenu les résultats suivants :

| exemple | Evaluation témoin A | Evaluation témoin B | Evaluation témoin C | Evaluation témoin D | moyenne |
|---|---|---|---|---|---|
| 1 (sans lavage) | 5 | 5 | 5 | 5 | 5 |
| 1a (1 cycle lavage) | 5 | 5 | 4 | 5 | 4,75 |
| 1b (2 cycles lavage) | 5 | 3 | 3 | 3 | 3,5 |
| 2 (sans lavage) | 1 | 4 | 2 | 1 | 2 |
| 2a (1 cycle lavage) | 3 | 3 | 5 | 3 | 3,5 |
| 2b (2 cycles lavage) | 4 | 2 | 1 | 3 | 2,5 |
| 3 (sans lavage) | 0 | 1 | 0 | 2 | 0,75 |
| 3a (1 cycle lavage) | 1 | 1 | 1 | 4 | 1,75 |
| 3b (2 cycles lavage) | 3 | 3 | 3 | 4 | 3,25 |
| 4 (sans lavage) | 0 | 0 | 1 | 0 | 0,25 |
| 4a (1 cycle lavage) | 0 | 0 | 0 | 1 | 0,25 |
| 4b (2 cycles lavage) | 0 | 0 | 0 | 0 | 0 |

Les résultats obtenus montrent que le stratum cornéum traité avec le polymère 1 et irradié avec la lumière (exemple 4) présente la plus faible odeur de sueur perçue par le panel. Ainsi, le traitement du stratum cornéum avec le polymère selon l'invention et avec l'exposition à la lumière confère une bonne propriété déodorante.
De même, après 1 cycle et 2 cycles de lavage, le stratum cornéum traité avec le polymère 1 présente la plus faible odeur de sueur perçue par le panel. Le traitement selon l'invention confère ainsi une action déodorante bien résistante à l'eau.

On a également évalué l'effet déodorant des polymères 2 à 5 selon le protocole de l'exemple 4 en utilisant respectivement les compositions 4 à 7 (exemples 5 à 8 respectivement) ; on a relevé les évaluations déodorantes après 2 cycles de lavage.

On a obtenu les résultats suivants :

| exemple | Evaluation témoin A | Evaluation témoin B | Evaluation témoin C | Evaluation témoin D | moyenne |
|---|---|---|---|---|---|
| 5 (Polymère 2) (2 cycles lavage) | 1 | 2 | 2 | 2 | 1,75 |
| 6 (Polymère 3) (2 cycles lavage) | 1 | 0 | 0 | 0 | 0,25 |
| 7 (Polymère 4) (2 cycles lavage) | 1 | 2 | 0 | 0 | 0,75 |
| 8 (Polymère 5) (2 cycles lavage) | 1 | 0 | 1 | 1 | 0,75 |

Les résultats obtenus montrent que le stratum corneum traité avec les polymères 2 à 5 et irradié avec la lumière présentent une faible odeur perçue par le panel.

### Exemple 9 :

On prépare un déodorant ayant la composition suivante :

| | |
|---|---|
| - polymère de l'exemple 1 | 1 g |
| - hydroxyéthyl cellulose (NATROSOL® 250 HHR CS de chez Ashland) | 0,2 g |
| - Conservateurs qs | |
| - Eau qsp | 100 g |

La composition obtenue est appliquée sur la peau des aisselles puis on irradie la surface de la peau traitée avec une lumière blanche (lampe Lite-Pad de la société Reicorp) pendant 5 minutes.

La composition appliquée sous les aisselles permet de diminuer les odeurs dues à la transpiration.

### Exemple 10 :

On prépare un déodorant ayant la composition suivante :

| | |
|---|---|
| - polymère de l'exemple 2 | 10 g |
| - hydroxyéthyl cellulose (NATROSOL® 250 HHR CS de chez Ashland) | 0,3 g |
| - Conservateurs qs | |
| - Eau qsp | 100 g |

La composition obtenue est appliquée sur la peau des aisselles puis on irradie la surface de la peau traitée avec une lumière bleue (goLITE BLU de la société Philips) pendant 5 minutes.

La composition appliquée sous les aisselles permet de diminuer les odeurs dues à la transpiration.

### Exemple 11 :

On prépare un déodorant ayant la composition suivante :

| | |
|---|---|
| - polymère de l'exemple 3 | 5 g |
| - hydroxyéthyl cellulose (NATROSOL® 250 HHR CS de chez Ashland) | 0,2 g |
| - Conservateurs qs | |
| - Eau qsp | 100 g |

La composition obtenue est appliquée sur la peau des aisselles puis on irradie la surface de la peau traitée avec une lumière bleue (lampe Camag Box 3 de la société Camag) pendant 1 minute.

Une composition similaire est faite en utilisant le polymère 4 ou le polymère 5 à la place du polymère 3.

La composition appliquée sous les aisselles permet de diminuer les odeurs dues à la transpiration.

### Exemple 12 :

On prépare une composition déodorante suivante :

| | |
|---|---|
| - polymère de l'exemple 3 | 5 g |
| - Conservateurs qs | |
| - Eau qsp | 100 g |

La composition obtenue est appliquée sur les semelles en liège de la société Sunbed (référence 550), on laisse sécher à température ambiante (25°C) pendant 30 minutes puis on irradie la surface des semelles avec une lumière bleue (lampe Camag Box 3 de la société Camag) pendant 1 minute.

La semelle ainsi traitée, disposée dans des chaussures, permet de diminuer les odeurs des pieds.

### Exemple 13 :

On prépare une composition déodorante suivante :

| | |
|---|---|
| - polymère de l'exemple 2 | 5 g |
| - Conservateurs qs | |
| - Eau qsp | 100 g |

Une pièce de coton (5 cm x 4 cm) (vendu par la société SDC Enterprises Limited, UK ; référence 1205), a été trompée dans la composition obtenue pendant 3 minutes puis on l'a laissé sécher à température ambiante (25°C) pendant 30 minutes. Ensuite on a irradié la surface du coton avec une lumière bleue (lampe Camag Box 3 de la société Camag) pendant 6 minutes (retournant la pièce de laine après 3 minutes). La composition appliquée sur le coton permet de diminuer les odeurs.
On peut ainsi traiter une pièce de tissu en coton de plus grande dimension pour ensuite, après traitement, confectionner un vêtement tel qu'un teeshirt. La personne habillée avec un tel tee-shirt peut ainsi désodoriser le corps pendant la journée.

## Revendications

1. Procédé cosmétique pour traiter les odeurs corporelles humaines en particulier les odeurs axillaires et éventuellement la transpiration humaine comprenant :
(i) une étape consistant à appliquer sur la peau une composition, notamment cosmétique, comprenant, dans un milieu physiologiquement acceptable, un polymère de chitosane dont les groupes amino sont greffés de groupements photoactif de type azide ou diazirine de formule (I) : 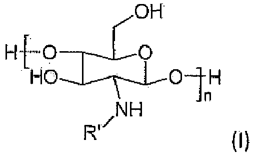
dans laquelle R' représente indépendamment H ou un groupement acétyle ou un groupement -CO-L-X , le polymère renfermant au moins un groupement R' = -CO-L-X ,
L étant un groupe divalent hydrocarboné comprenant de 1 à 20 atomes de carbone, de préférence de 2 à 10 atomes de carbone, linéaire, ramifiée ou cyclique, saturé ou insaturé, et pouvant être interrompu par un ou plusieurs hétéroatomes non adjacents choisis parmi l'oxygène, le soufre ou groupements -NH-, -CO-, -CONH-, -COO-, -O-CO-N(Ra)-, en particulier -O-CO-NH-, -N(Rb)-CO-N(Rc)-, en particulier -NH-CO-NH-, ledit groupe divalent pouvant éventuellement être substitué pour un ou plusieurs groupes choisi parmi les groupes hydroxyle, amine, acide carboxylique, amide, cyano, acyl(C₁-C₄)amino,
avec Ra, Rb, Rc désignant indépendamment un atome d'hydrogène ou un radical alkyle C₁-C₆ linéaire ou ramifié , de préférence linéaire ;
X désigne un groupe photoactif de type azide ou diazirine ;
n varie de 5 à 2000 ;
(ii) une étape consistant à exposer la peau à un rayonnement lumineux, de préférence pendant au moins 5 seconde ;
l'étape consistant à appliquer un rayonnement lumineux étant effectuée après ou en même temps que l'étape consistant à appliquer la composition cosmétique comprenant le chitosane greffé (I).

2. Procédé selon la revendication précédente, **caractérisé en ce que** le chitosane greffé (I) a un taux molaire de greffage en groupements photoactifs -CO-L-X allant de 0,1 à 50 %, de préférence allant de 1 à 30 %, et préférentiellement allant de 1 à 20 %.

3. Procédé selon l'une des revendications précédentes, **caractérisé en ce que** L est un radical divalent hydrocarboné en C₁-C₂₀, de préférence en C₁-C₁₀ linéaire, ramifié ou cyclique, saturé ou insaturé (dont aromatique), pouvant être interrompu par un ou plusieurs hétéroatomes non adjacents choisi parmi l'oxygène ou par un ou plusieurs groupements non adjacents choisis parmi -NH-, -CO-, - O-CO-, -NH-CO-.

4. Procédé selon l'une des revendications précédentes, **caractérisé en ce que** le chitosane greffé (I) a un degré d'acétylation (-CD=CH₃) allant de 0 à 50 %, de préférence allant de 5 à 30 %, et plus préférentiellement allant de 10 à 30 %.

5. Procédé selon l'une des revendications précédentes, **caractérisé en ce que** n varie de 5 à 280, de préférence de 15 à 165.

6. Procédé selon l'une des revendications précédentes, **caractérisé en ce que** le polymère de chitosane greffé est de formule (Ia) : 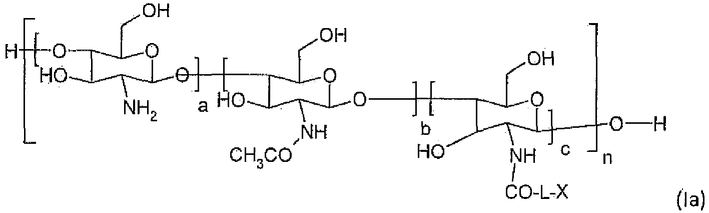
dans laquelle le chitosane greffé comprend a motifs portant un groupe amino libre, b motifs portant un groupe amino N-acétylé et c motifs portant un groupe amino greffé d'un groupe photoactif -CO-L-X ;
b va de 0 à 0,5, de préférence de 0,05 à 0,30, et mieux de 0,1 à 0,30 ;
c va de 0,001 à 0,5, de préférence de 0,01 à 0,3 , et mieux de 0,01 à 0,2 ;
et a + b + c = 1
X , L et n ayant les significations selon la revendication 1.

7. Procédé selon l'une des revendications précédentes, **caractérisé en ce que** L est choisi parmi les groupes suivants :

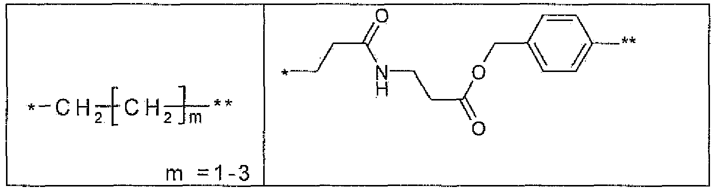

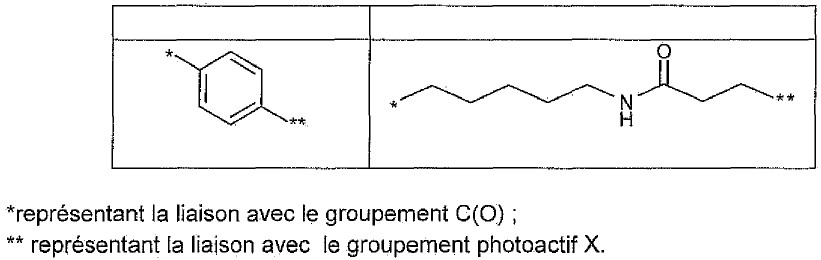

8. Procédé selon l'une des revendications précédentes, **caractérisé en ce que** le groupe photoactif X peut être choisi parmi les groupes suivants : 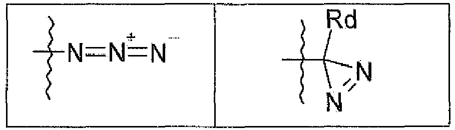 avec Rd désignant H ou un radical alkyle en C₁-C₆ , linéaire ou ramifié, de préférence linéaire, ou un radical CF₃ ;
de préférence choisi parmi :

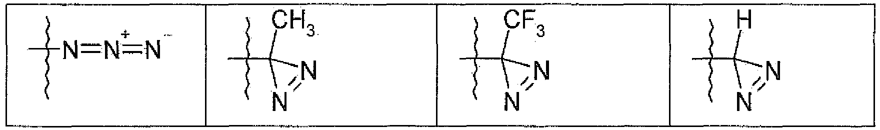

9. Procédé selon l'une des revendications précédentes, **caractérisé en ce que** le groupement -CO-L-X est choisi parmi : 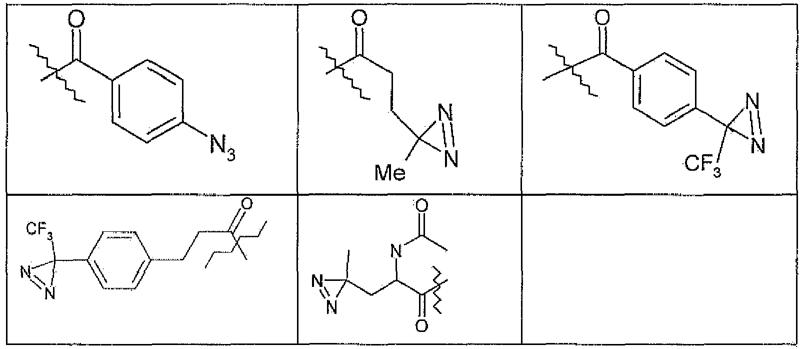 et de préférence choisi parmi :

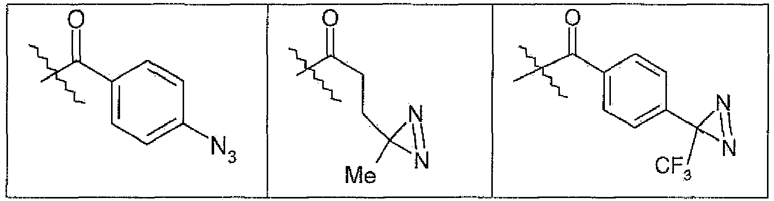

10. Procédé selon l'une des revendications précédentes, **caractérisé en ce que** le chitosane greffé (I) ou (la) est présent dans la composition en une teneur allant de 0,1 à 10 % en poids, par rapport au poids total de la composition, de préférence allant de 0,5 % à 10 % en poids, et préférentiellement allant de 1 à 8 % en poids, et plus préférentiellement allant de 1 % à 6 % en poids.

11. Procédé selon l'une quelconque des revendications précédentes, dans lequel l'étape (ii) consistant à appliquer un rayonnement lumineux est effectuée après l'étape (i) consistant à appliquer la composition cosmétique comprenant le chitosane greffé (I).

12. Procédé selon l'une quelconque des revendications précédentes, **caractérisé en ce que** le rayonnement lumineux est la lumière naturelle ou à la lumière artificielle de longueur d'onde comprise entre 360 et 600 nm.

13. Procédé selon l'une quelconque des revendications précédentes, dans lequel le rayonnement lumineux a une source choisie parmi les lampes d'arc telles que les lampes à xénon et les lampes à mercure; les lampes à fluorescence; les lampes à incandescence telles que les halogènes, les LEDs et les lasers.

14. Procédé selon l'une quelconque l'une des revendications précédentes, dans lequel la durée d'exposition au rayonnement lumineux est d'au moins 5 secondes, de préférence cette durée d'exposition peut aller de 10 secondes à 15 minutes, notamment entre 15 secondes et 10 minutes, encore mieux entre 20 secondes et 5 minutes.

15. Polymère de formule (II) : 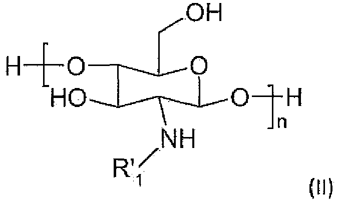 dans laquelle R'₁ représente indépendamment H ou un groupement acétyle ou un groupement -CO-L-X de formule (c) suivante : 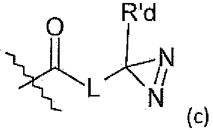 dans laquelle :
L étant un groupe divalent hydrocarboné comprenant de 1 à 20 atomes de carbone, de préférence de 2 à 10 atomes de carbone, linéaire, ramifiée ou cyclique, saturé ou insaturé, et pouvant être interrompu par un ou plusieurs hétéroatomes non adjacents choisis parmi l'oxygène, le soufre ou groupements -NH-, -CO-, -CONH-, -COO-, -O-CO-N(Ra)-, en particulier -O-CO-NH-, -N(Rb)-CO-N(Rc)-, en particulier -NH-CO-NH-, ledit groupe divalent pouvant éventuellement être substitué pour un ou plusieurs groupes choisi parmi les groupes hydroxyle, amine, acide carboxylique, amide, cyano, acyl(C₁-C₄)amino,
avec Ra, Rb, Rc désignant indépendamment un atome d'hydrogène ou un radical alkyle C₁-C₆ linéaire ou ramifié , de préférence linéaire ;
de préférence, L est un radical divalent hydrocarboné en C₁-C₂₀, linéaire, ramifié ou cyclique, saturé ou insaturé (dont aromatique), pouvant être interrompu par un ou plusieurs hétéroatomes non adjacents choisi parmi l'oxygène ou par un ou plusieurs groupements non adjacents choisis parmi -NH-, -CO-, - O-CO-, -NH-CO- ;
R'd désigne H ou un radical alkyle en C₁-C₄ linéaire ou ramifié, de préférence linéaire, ou un radical CF₃; de préférence R'd est choisi parmi un atome d'hydrogène, un radical méthyle, un radical CF₃ ;
le polymère renfermant au moins un groupement -CO-L-X de formule (c),
n varie de 5 à 1700, de préférence de 5 à 280, plus préférentiellement de 15 à 165.

16. Polymère selon la revendication précédente, **caractérisé en ce que** le chitosane greffé (I) a un taux molaire de greffage en groupements photoactifs -CO-L-X allant de 0,1 à 50 %, de préférence allant de 1 à 30 %, et préférentiellement allant de 1 à 20 %.

17. Polymère selon l'une des revendications 15 ou 16, **caractérisé en ce que** le chitosane greffé (I) a un degré d'acétylation (-CO-CH₃) allant de 0 à 50 %, de préférence allant de 5 à 30 %, et plus préférentiellement allant de 10 à 30 %.

18. Polymère selon l'une des revendications 15 à 17, **caractérisé en ce qu'**il est de formule (II') : 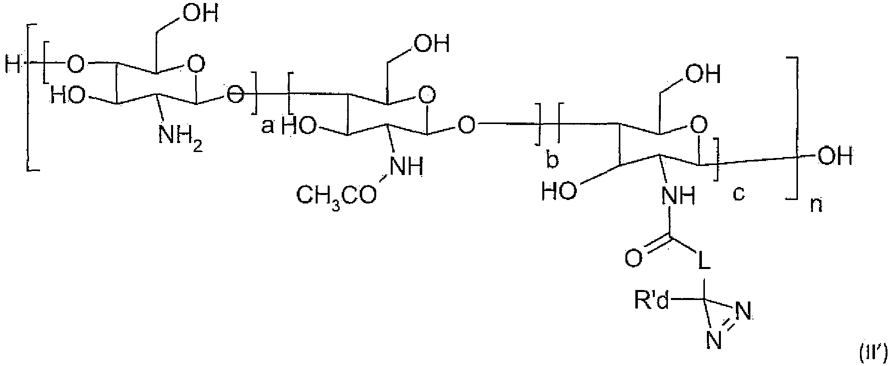 dans laquelle
b va de 0 à 0,5, de préférence de 0,05 à 0,30, et mieux de 0,1 à 0,30 ;
c va de 0,001 à 0,5 , de préférence de 0,01 à 0,3 , et mieux de 0,01 à 0,2 ;
et a + b + c = 1
L, R'd ayant les significations selon la revendication 15.

19. Polymère selon l'une des revendications 15 à 18 **caractérisé en ce que** le polymère de chitosane greffé (II) est de formule (IIa): 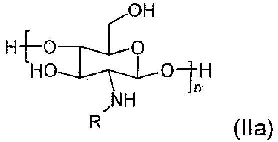 dans laquelle R désigne H ou acétyle ou un groupement (J) : 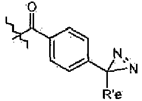 dans lequel R'e = CH₃ ou CF₃, le polymère comprenant au moins un groupement (J) ;
ou de formule (IIb) 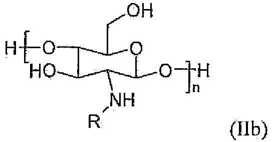 dans laquelle R désigne H ou acétyle ou un groupement (K) : 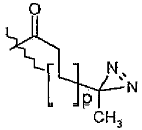 avec p= 1 à 3, le polymère comprenant au moins un groupement (K).

20. Composition comprenant, dans un milieu physiologiquement acceptable un polymère (II) ou (II') ou (IIa) ou (IIb) selon l'une des revendications 15 à 19.

21. Composition selon la revendication précédente, **caractérisée en ce que** le polymère (II) ou (IIa) ou (IIb) est présent en une teneur allant de 0,1 à 10 % en poids, par rapport au poids total de la composition, de préférence allant de 0,5 % à 10 % en poids, et préférentiellement allant de 1 à 8 % en poids, et plus préférentiellement allant de 1 % à 6 % en poids.

22. Composition selon la revendication 20 ou 21, **caractérisée en ce qu'**elle comprend un adjuvant cosmétique choisi parmi l'eau, les émulsionnants, les conservateurs, les séquestrants, les parfums, les épaississants, les huiles, les cires, les polymères filmogènes.

23. Procédé pour désodoriser les pieds et/ou les chaussures consistant à disposer dans une chaussure une semelle dont la surface a été traitée par application sur la dite surface d'une composition comprenant un chitosane greffé (I) selon l'une des revendications 1 à 9 puis l'exposition de la surface traitée à un rayonnement lumineux, de préférence pendant au moins 5 secondes, selon l'une des revendications 1 et 12 à 14.

24. Procédé pour désodoriser le corps (ou partie du corps) consistant à habiller le corps (ou partie du corps) avec des vêtements en tissu textile dont la surface a été traitée par application sur la surface du textile d'une composition comprenant un chitosane greffé (I) selon l'une des revendications 1 à 9 puis d'exposer la surface traitée à un rayonnement lumineux, de préférence pendant au moins 5 secondes, selon l'une des revendications 1 et 12 à 14.

25. Procédé selon la revendication précédente **caractérisé en ce que** la partie du corps est les pieds et le vêtement textile est choisi parmi les chaussettes, les bas, le collant.

## Patentansprüche

1. Kosmetisches Verfahren zur Behandlung von menschlichen Körpergerüchen, insbesondere Gerüchen der Achselhöhlen und möglicherweise des menschlichen Schweißes, umfassend:
(i) einen Schritt, der darin besteht, auf die Haut eine Zusammensetzung, insbesondere kosmetischer Art, aufzubringen, welche ein Chitosanpolymer umfasst, das in einem physiologisch unbedenklichen Medium vorliegt, wobei seine Aminogruppen mit photoaktiven Pfropfgruppen vom Azid- oder Diazirintyp nach Formel (I) versehen sind: 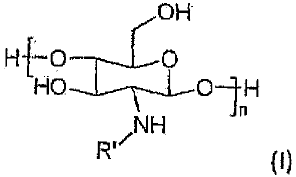
wobei R' auf unabhängige Weise für H oder eine Acetylgruppe oder eine -CO-L-X-Gruppe steht, wobei das Polymer mindestens eine Gruppe R' = -CO-L-X beinhaltet,
L eine zweibindige Kohlenwasserstoffgruppe geradkettiger, verzweigter oder zyklischer, gesättigter oder ungesättigter Art ist, die 1 bis 20 Kohlenstoffatome, vorzugsweise 2 bis 10 Kohlenwasserstoffatome, umfasst, wobei sie von einem oder mehreren nicht-benachbarten Heteroatomen, welche aus Sauerstoff und Schwefel ausgewählt sind, oder den Gruppen -NH-, -CO-, -CONH-, -COO-, -O-CO-N(Ra)-, insbesondere -O-CO-NH-, -N(Rb)-CO-N(Rc)-, insbesondere -NH-CO-NH-, unterbrochen sein kann, wobei die zweibindige Gruppe möglicherweise mit einer oder mehreren Gruppen substituiert sein kann, die aus den Gruppen Hydroxyl, Amin, Carbonsäure, Amid, Cyano, Acyl (C₁-C₄) amino ausgewählt sind,
wobei Ra, Rb, Rc unabhängig voneinander ein Wasserstoffatom oder einen geradkettigen oder verzweigten C₁-C₆-Alkylrest, vorzugsweise geradkettiger Art, bezeichnen;
X eine photoaktive Gruppe vom Azin- oder Diazirintyp bezeichnet;
n im Bereich von 5 bis 2.000 liegen kann;
(ii) einen Schritt des Einwirkenlassens einer Lichtstrahlung auf die Haut, vorzugsweise für eine Dauer von mindestens 5 Sekunden;
wobei des Schritt des Einwirkenlassens einer Lichtstrahlung nach oder gleichzeitig mit dem Schritt des Aufbringens der kosmetischen Zusammensetzung, welche das gepfropfte Chitosan (I) umfasst, durchgeführt wird.

2. Verfahren gemäß dem vorherigen Anspruch, **dadurch gekennzeichnet, dass** das gepfropfte Chitosan (I) einen molaren Pfropfanteil an photoaktiven -CO-L-X-Gruppen aufweist, der im Bereich von 0,1 bis 50 %, vorzugsweise von 1 bis 30 % und bevorzugt von 1 bis 20 % liegt.

3. Verfahren gemäß einem der vorhergehenden Ansprüche, **dadurch gekennzeichnet, dass** L ein zweibindiger Kohlenwasserstoffrest von C₁-C₂₀, vorzugsweise von C₁-C₁₀, geradkettiger, verzweigter oder zyklischer, gesättigter oder ungesättigter (einschließlich aromatischer) Art ist, wobei er von einem oder mehreren nicht-benachbarten Heteroatomen, die aus Sauerstoff ausgewählt sind, oder von einer oder mehreren nicht-benachbarten Gruppen, die aus -NH-, -CO-, -O-CO-, -NH-CO- ausgewählt sind, unterbrochen sein kann.

4. Verfahren gemäß einem der vorhergehenden Ansprüche, **dadurch gekennzeichnet, dass** das gepfropfte Chitosan (I) einen Acetylierungsgrad (-CO-CH₃) aufweist, der im Bereich von 0 bis 50 %, vorzugsweise im Bereich von 5 bis 30 %, und stärker bevorzugt im Bereich von 10 bis 3 0 % liegt.

5. Verfahren gemäß einem der vorhergehenden Ansprüche, **dadurch gekennzeichnet, dass** n im Bereich von 5 bis 280, vorzugsweise von 15 bis 165, liegen kann.

6. Verfahren gemäß einem der vorhergehenden Ansprüche, **dadurch gekennzeichnet, dass** das gepfropfte Chitosanpolymer der Formel (Ia) entspricht: 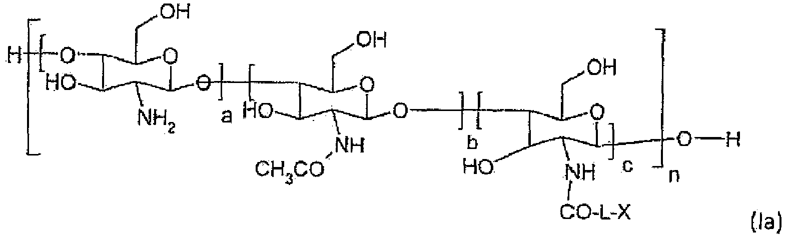
wobei das gepfropfte Chitosan a Bausteine umfasst, die mit einer freie Aminogruppe versehen sind, b Bausteine, die mit einer N-acetylierten Aminogruppe versehen sind, und c Bausteine, die mit einer Aminogruppe versehen sind, welche eine photoaktive -CO-L-X Pfropfgruppe aufweist;
b im Bereich von 0 bis 0,5, vorzugsweise von 0,05 bis 0,30, und besser von 0,1 bis 0,30 liegt;
c im Bereich von 0,001 bis 0,5, vorzugsweise von 0,01 bis 0,3, und besser von 0,01 bis 0,2 liegt;
und a + b + c = 1 ist
X, L und n die Bedeutungen gemäß Anspruch 1 haben.

7. Verfahren gemäß einem der vorhergehenden Ansprüche, **dadurch gekennzeichnet, dass** L aus den folgenden Gruppen ausgewählt ist:

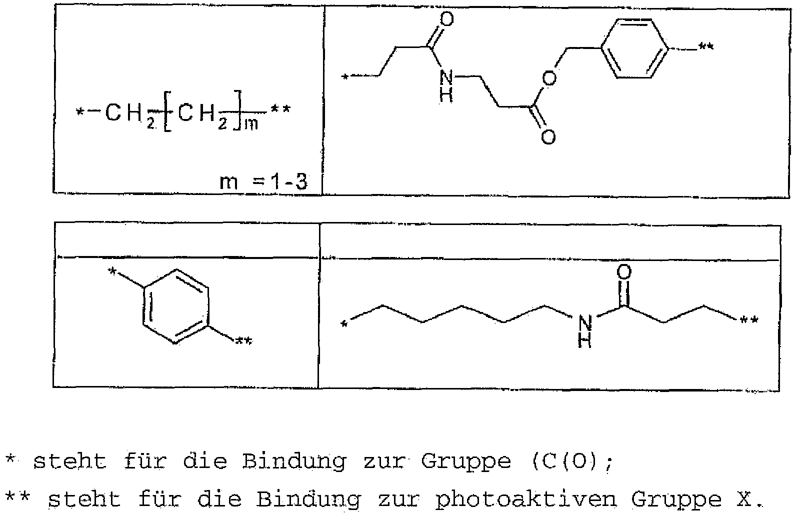

8. Verfahren gemäß einem der vorhergehenden Ansprüche, **dadurch gekennzeichnet, dass** die photoaktive Gruppe X aus den folgenden Gruppen ausgewählt sein kann: 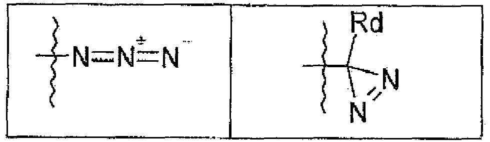 wobei Rd für H oder einen C₁-C₆-Alkylrest geradkettiger oder verzweigter Art, vorzugsweise geradkettiger Art, oder einen CF₃-Rest steht;
wobei sie vorzugsweise aus den folgenden ausgewählt ist:

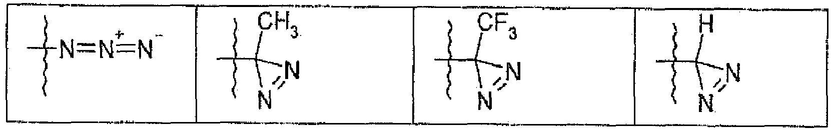

9. Verfahren gemäß einem der vorhergehenden Ansprüche, **dadurch gekennzeichnet, dass** die -CO-L-X-Gruppe aus den folgenden ausgewählt ist: 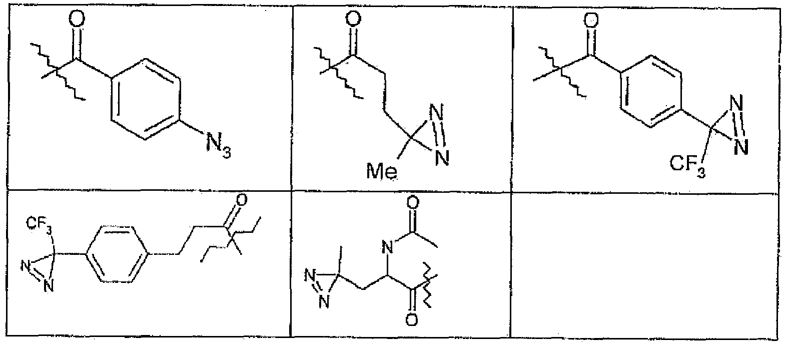 wobei sie vorzugsweise aus den folgenden ausgewählt ist:

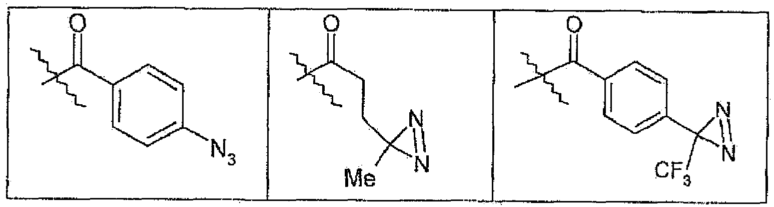

10. Verfahren gemäß einem der vorhergehenden Ansprüche, **dadurch gekennzeichnet, dass** das gepfropfte Chitosan (I) oder (Ia) in der Zusammensetzung zu einem Gehalt im Bereich von 0,1 bis 10 Gewichts-%, bezogen auf das Gesamtgewicht der Zusammensetzung, vorzugsweise von 0,5 bis 10 Gewichts-%, und bevorzugt von 1 bis 8 Gewichts-%, und stärker bevorzugt von 1 bis 6 Gewichts-%, vorliegt.

11. Verfahren gemäß einem beliebigen der vorhergehenden Ansprüche, wobei der Schritt (ii), welcher darin besteht, eine Lichtstrahlung zur Anwendung zu bringen, nach dem Schritt (i) durchgeführt wird, welcher darin besteht, die kosmetische Zusammensetzung aufzubringen, welche das gepfropfte Chitosan (I) umfasst.

12. Verfahren gemäß einem beliebigen der vorhergehenden Ansprüche, **dadurch gekennzeichnet, dass** es sich bei der Lichtstrahlung um natürliches Licht oder künstliches Licht mit einer Wellenlänge im Bereich von 360 bis 600 nm ist.

13. Verfahren gemäß einem beliebigen der vorhergehenden Ansprüche, wobei die Lichtstrahlung eine Quelle hat, die aus den Gasentladungslampen etwa den Xenonlampen oder den Quecksilberdampflampen; den Fluoreszenzlampen; den Glühlampen wie etwa den Halogenlampen, den LEDs und den Lasern ausgewählt ist.

14. Verfahren gemäß einem beliebigen der vorhergehenden Ansprüche, wobei die Einwirkungsdauer des Lichtstrahlung mindestens 5 Sekunden beträgt, wobei diese Einwirkungsdauer vorzugsweise im Bereich von 10 Sekunden bis 15 Minuten, insbesondere von 15 Sekunden bis 10 Minuten, noch besser von 20 Sekunden bis 5 Minuten, liegen kann.

15. Polymer nach Formel (II): 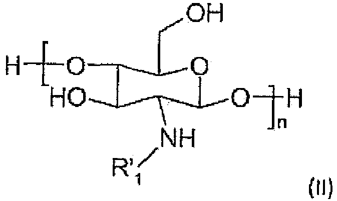 wobei R'₁ auf unabhängige Weise für H oder eine Acetylgruppe oder eine -CO-L-X-Gruppe nach der folgenden Formel (c) steht: 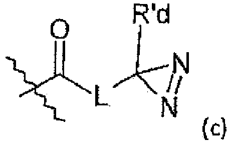 wobei:
L eine zweibindige Kohlenwasserstoffgruppe geradkettiger, verzweigter oder zyklischer, gesättigter oder ungesättigter Art ist, die 1 bis 20 Kohlenstoffatome, vorzugsweise 2 bis 10 Kohlenwasserstoffatome, umfasst, wobei sie von einem oder mehreren nicht-benachbarten Heteroatomen, welche aus Sauerstoff und Schwefel ausgewählt sind, oder den Gruppen -NH-, -CO-, -CONH-, -COO-, -O-CO-N(Ra)-, insbesondere - O-CO-NH-, -N(Rb)-CO-N(Rc)-, insbesondere -NH-CO-NH-, unterbrochen sein kann,
wobei die zweibindige Gruppe möglicherweise mit einer oder mehreren Gruppen substituiert sein kann, die aus den Gruppen Hydroxyl, Amin, Carbonsäure, Amid, Cyano, Acyl (C₁-C₄)amino ausgewählt sind,
wobei Ra, Rb, Rc unabhängig voneinander ein Wasserstoffatom oder einen geradkettigen oder verzweigten C₁-C₆-Alkylrest, vorzugsweise geradkettiger Art, bezeichnen;
wobei L vorzugsweise ein zweibindiger C₁-C₂₀-Kohlenwasserstoffrest geradkettiger, verzweigter oder zyklischer, gesättigter oder ungesättigter (einschließlich aromatischer) Art ist, wobei er von einem oder mehreren nicht-benachbarten Heteroatomen, die aus Sauerstoff ausgewählt sind, oder von einer oder mehreren nicht-benachbarten Gruppen, die aus -NH-, -CO-, - O-CO-, -NH-CO- ausgewählt sind, unterbrochen sein kann; R'd für H oder einen geradkettigen oder verzweigten C₁-C₄-Alkylrest, vorzugsweise geradkettiger Art, oder einen CF₃-Rest steht, wobei R'd vorzugsweise aus einem Wasserstoffatom, einem Methylrest, einem CF₃-Rest ausgewählt ist;
wobei das Polymer mindestens eine -CO-L-X-Gruppe nach Formel (c) beinhaltet,
n im Bereich von 5 bis 1.700, vorzugsweise von 5 bis 280, stärker bevorzugt von 15 bis 165, liegen kann.

16. Polymer nach dem vorherigen Anspruch, **dadurch gekennzeichnet, dass** das gepfropfte Chitosan (I) einen molaren Pfropfanteil an photoaktiven -CO-L-X-Gruppen aufweist, der im Bereich von 0,1 bis 50 %, vorzugsweise von 1 bis 30 % und bevorzugt von 1 bis 20 % liegt.

17. Polymer gemäß einem der Ansprüche 15 oder 16, **dadurch gekennzeichnet, dass** das gepfropfte Chitosan (I) einen Acetylierungsgrad (-CO-CH₃) aufweist, der im Bereich von 0 bis 50 %, vorzugsweise im Bereich von 5 bis 30 %, und stärker bevorzugt im Bereich von 10 bis 30 % liegt.

18. Polymer nach einem der Ansprüche 15 bis 17, **dadurch gekennzeichnet, dass** es der Formel (II') entspricht: 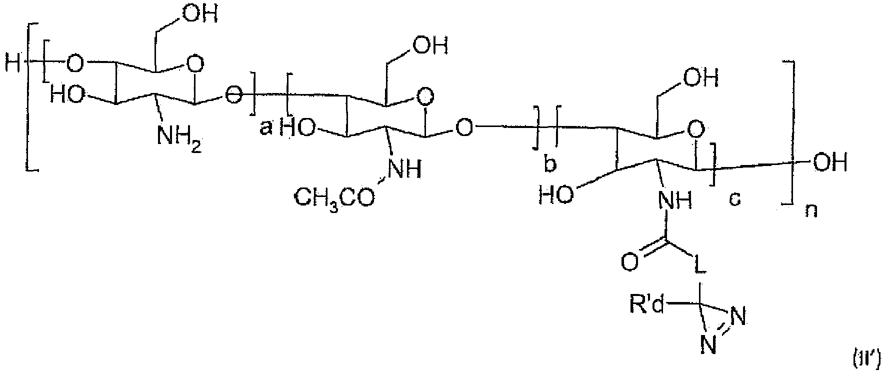 wobei
b im Bereich von 0 bis 0,5, vorzugsweise von 0,05 bis 0,30, und besser von 0,1 bis 0,30 liegt;
c im Bereich von 0,001 bis 0,5, vorzugsweise von 0,01 bis 0,3, und besser von 0,01 bis 0,2 liegt;
und a + b + c = 1 ist
wobei L, R'd die Bedeutungen gemäß Anspruch 15 haben.

19. Polymer gemäß einem der Ansprüche 15 bis 18, **dadurch gekennzeichnet, dass** das gepfropfte Chitosanpolymer (II) der Formel (IIa) entspricht: 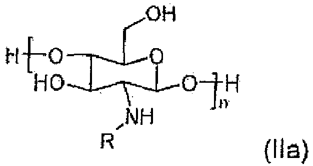 in welcher R für H oder Acetyl oder eine Gruppe (J) : 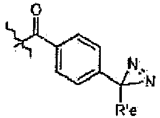 steht, in welcher
R'e = CH₃ oder CF₃ ist, wobei das Polymer mindestens eine Gruppe (J) umfasst;
oder der Formel (IIb) 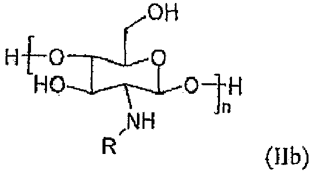 in welcher R für H oder Acetyl oder eine Gruppe (K) : 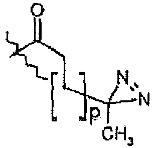 steht, mit p = 1 bis 3, wobei das Polymer mindestens eine Gruppe (K) umfasst.

20. Zusammensetzung, die in einem physiologisch unbedenklichen Medium ein Polymer (II) oder (II') oder (IIa) oder (IIb) gemäß einem der Ansprüche 15 bis 19 umfasst.

21. Zusammensetzung gemäß dem vorhergehenden Anspruch, **dadurch gekennzeichnet, dass** das Polymer (II) oder (IIa) oder (IIb) zu einem Gehalt im Bereich von 0,1 bis 10 Gewichts-%, bezogen auf das Gesamtgewicht der Zusammensetzung, vorzugsweise von 0,5 bis 10 Gewichts-%, und bevorzugt von 1 bis 8 Gewichts-%, und stärker bevorzugt von 1 bis 6 Gewichts-% vorliegt.

22. Zusammensetzung gemäß Anspruch 20 oder 21, **dadurch gekennzeichnet, dass** sie einen kosmetischen Hilfsstoff umfasst, der aus Wasser, Emulgatoren, Konservierungsstoffen, Komplexierungsmitteln, Duftstoffen, Verdickungsmitteln, Ölen, Wachsen, filmbildenden Polymeren ausgewählt ist.

23. Verfahren zum Desodorisieren der Füße und/oder der Schuhe, welches darin besteht, in einem Schuh eine Sohle anzuordnen, deren Oberfläche behandelt wurde, indem auf die Oberfläche eine Zusammensetzung aufgebracht wurde, die ein gepfropftes Chitosan (I) gemäß einem der Ansprüche 1 bis 9 umfasst, woraufhin die behandelte Oberfläche gemäß einem der Ansprüche 1 und 12 bis 14 einer Lichtstrahlung ausgesetzt wird, vorzugsweise für eine Dauer von mindestens 5 Sekunden.

24. Verfahren zur Desodorisieren des Körpers (oder von Körperteilen), welches darin besteht, den Körper (oder Körperteile) mit Kleidungsstücken aus einem Textilstoff zu bekleiden, deren Oberfläche behandelt wurde, indem auf die Oberfläche der Textilie eine Zusammensetzung aufgebracht wurde, die ein gepfropftes Chitosan (I) gemäß einem der Ansprüche 1 bis 9 umfasst, woraufhin die behandelte Oberfläche gemäß einem der Ansprüche 1 und 12 bis 14 einer Lichtstrahlung ausgesetzt wird, vorzugsweise für eine Dauer von mindestens 5 Sekunden.

25. Verfahren gemäß dem vorhergehenden Anspruch, **dadurch gekennzeichnet, dass** es sich bei dem Körperteil um die Füße handelt und das Textilkleidungsstück aus Socken, Nylonstrümpfen und -strumpfhosen ausgewählt ist.

## Claims

1. Cosmetic process for treating human body odor, in particular axillary odor, and optionally human perspiration, comprising:
(i) a step consisting in applying to the skin a composition, in particular a cosmetic composition, comprising, in a physiologically acceptable medium, a chitosan polymer in which the amino groups are grafted with photoactive groups of azide or diazirine type of formula (I): 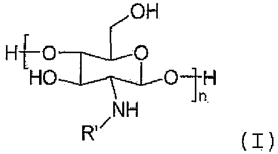
wherein R' independently represents H or an acetyl group or a group -CO-L-X, the polymer containing at least one group R' = -CO-L-X,
L being a linear, branched or cyclic, saturated or unsaturated divalent hydrocarbon-based group comprising from 1 to 20 carbon atoms, preferably from 2 to 10 carbon atoms, which may be interrupted with one or more non-adjacent heteroatoms chosen from sulfur, oxygen, or -NH-, -CO-, -CONH-, -COO-, -O-CO-N(Ra)-, in particular -O-CO-NH- or -N(Rb)-CO-N(Rc), in particular -NH-CO-NH-, groups, said divalent group possibly being substituted with one or more groups chosen from hydroxyl, amine, carboxylic acid, amide, cyano and (C₁-C₄) acylamino groups,
with Ra, Rb and Rc independently denoting a hydrogen atom or a linear or branched, preferably linear, C₁-C₆ alkyl radical;
X denotes a photoactive group of azide or diazirine type; n ranges from 5 to 2000;
(ii) a step consisting in exposing the skin to light radiation, preferably for at least 5 seconds;
the step consisting in applying light radiation being performed after or at the same time as the step consisting in applying the cosmetic composition comprising the grafted chitosan (I).

2. Process according to the preceding claim, **characterized in that** the grafted chitosan (I) has a molar degree of grafting with photoactive groups -CO-L-X ranging from 0.1% to 50%, preferably ranging from 1% to 30%, and preferentially ranging from 1% to 20%.

3. Process according to either of the preceding claims, **characterized in that** L is a linear, branched or cyclic, saturated or unsaturated (including aromatic) C₁-C₂₀, preferably C₁-C₁₀, divalent hydrocarbon-based radical, which may be interrupted with one or more non-adjacent heteroatoms chosen from oxygen or with one or more non-adjacent groups chosen from -NH-, -CO-, -O-CO- and -NH-CO-.

4. Process according to one of the preceding claims, **characterized in that** the grafted chitosan (I) has a degree of acetylation (-CO-CH₃) ranging from 0% to 50%, preferably ranging from 5% to 30%, and more preferentially ranging from 10% to 30%.

5. Process according to one of the preceding claims, **characterized in that** n ranges from 5 and 280 and preferably from 15 to 165.

6. Process according to one of the preceding claims, **characterized in that** the grafted chitosan polymer is of formula (Ia): 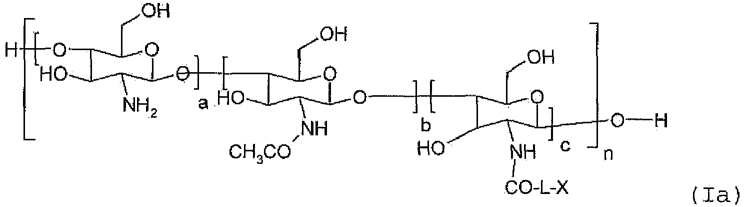
wherein the grafted chitosan comprises a units bearing a free amino group, b units bearing an N-acetyl amino group and c units bearing an amino group grafted with a photoactive group -CO-L-X;
b ranges from 0 to 0.5, preferably from 0.05 to 0.30, and better still from 0.1 to 0.30;
c ranges from 0.001 to 0.5, preferably from 0.01 to 0.3, and better still from 0.01 to 0.2;
and a + b + c = 1.
X, L and n having the meanings according to Claim 1.

7. Process according to one of the preceding claims, **characterized in that** L is chosen from the following groups:

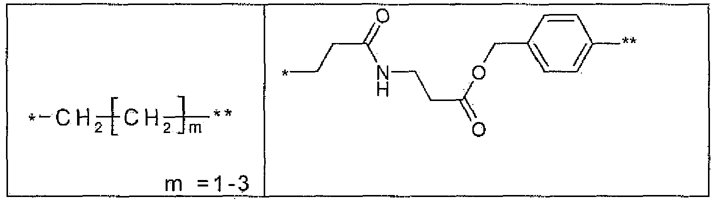

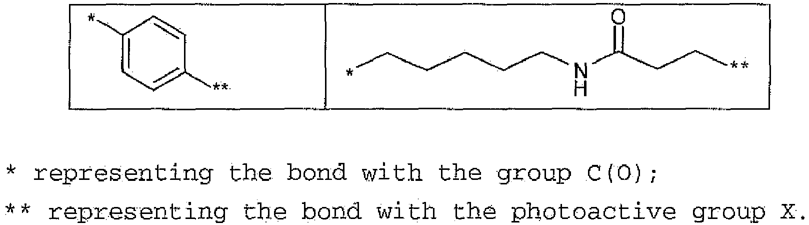

8. Process according to one of the preceding claims, **characterized in that** photoactive group X may be chosen from the following groups: 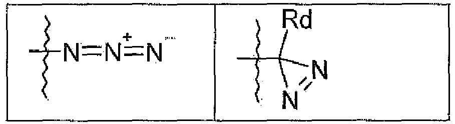 with Rd denoting H or a linear or branched, preferably linear, C₁-C₆ alkyl radical, or a CF₃ radical;
preferably chosen from:

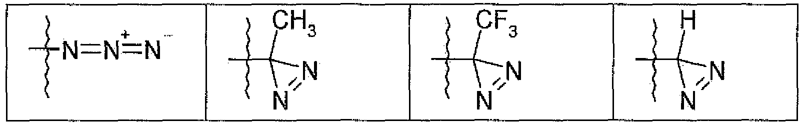

9. Process according to one of the preceding claims, **characterized in that**
the group -CO-L-X is chosen from: 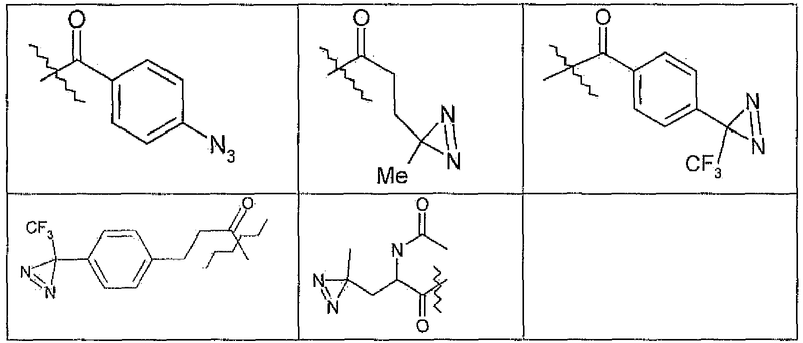 and preferably chosen from:

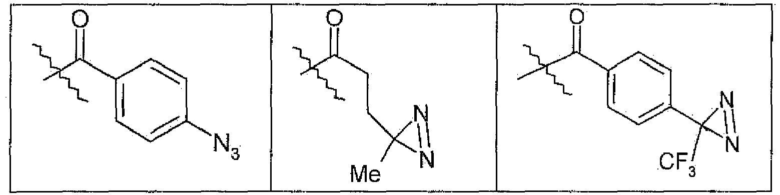

10. Process according to one of the preceding claims, **characterized in that** the grafted chitosan (I) or (Ia) is present in the composition in a content ranging from 0.1% to 10% by weight, relative to the total weight of the composition, preferably ranging from 0.5% to 10% by weight, preferentially ranging from 1% to 8% by weight, and more preferentially ranging from 1% to 6% by weight.

11. Process according to any one of the preceding claims, wherein step (ii) consisting in applying light radiation is performed after step (i) consisting in applying the cosmetic composition comprising the grafted chitosan (I) .

12. Process according to any one of the preceding claims, **characterized in that** the light radiation is natural light or artificial light with a wavelength of between 360 and 600 nm.

13. Process according to any one of the preceding claims, in which the light radiation has a source chosen from arc lamps such as xenon lamps and mercury lamps; fluorescent lamps; incandescent lamps such as halogen lamps; LEDs and lasers.

14. Process according to any one of the preceding claims, in which the exposure time to the light radiation is at least 5 seconds, and this exposure time may preferably range from 10 seconds to 15 minutes, in particular between 15 seconds and 10 minutes and better still between 20 seconds and 5 minutes.

15. Polymer of formula (II): 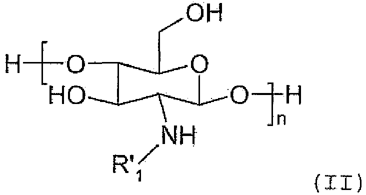 wherein R'₁ independently represents H or an acetyl group or a group -CO-L-X of formula (c) below: 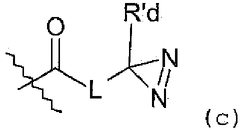 wherein:
L being a linear, branched or cyclic, saturated or unsaturated divalent hydrocarbon-based group comprising from 1 to 20 carbon atoms, preferably from 2 to 10 carbon atoms, which may be interrupted with one or more non-adjacent heteroatoms chosen from sulfur, oxygen, or -NH-, -CO-, -CONH-, -COO-, -O-CO-N(Ra)-, in particular -O-CO-NH- or -N(Rb)-CO-N(Rc), in particular -NH-CO-NH-, groups, said divalent group possibly being substituted with one or more groups chosen from hydroxyl, amine, carboxylic acid, amide, cyano and (C₁-C₄) acylamino groups,
with Ra, Rb and Rc independently denoting a hydrogen atom or a linear or branched, preferably linear, C₁-C₆ alkyl radical;
preferably, L is a linear, branched or cyclic, saturated or unsaturated (including aromatic) C₁-C₂₀ divalent hydrocarbon-based radical, which may be interrupted with one or more non-adjacent heteroatoms chosen from oxygen or with one or more non-adjacent groups chosen from -NH-, -CO-, -O-CO- and -NH-CO-;
R'd denotes H or a linear or branched, preferably linear, C₁-C₄ alkyl radical, or a CF₃ radical; preferably R'd is chosen from a hydrogen atom, a methyl radical and a CF₃ radical;
the polymer containing at least one group -CO-L-X of formula (c),
n ranges from 5 to 1700, preferably from 5 to 280 and more preferentially from 15 to 165.

16. Polymer according to the preceding claim, **characterized in that** the grafted chitosan (I) has a molar degree of grafting with photoactive groups -CO-L-X ranging from 0.1% to 50%, preferably ranging from 1% to 30%, and preferentially ranging from 1% to 20%.

17. Polymer according to either of Claims 15 and 16, **characterized in that** the grafted chitosan (I) has a degree of acetylation (-CO-CH₃) ranging from 0% to 50%, preferably ranging from 5% to 30%, and more preferentially ranging from 10% to 30%.

18. Polymer according to one of Claims 15 to 17, **characterized in that** it is of formula (II'): 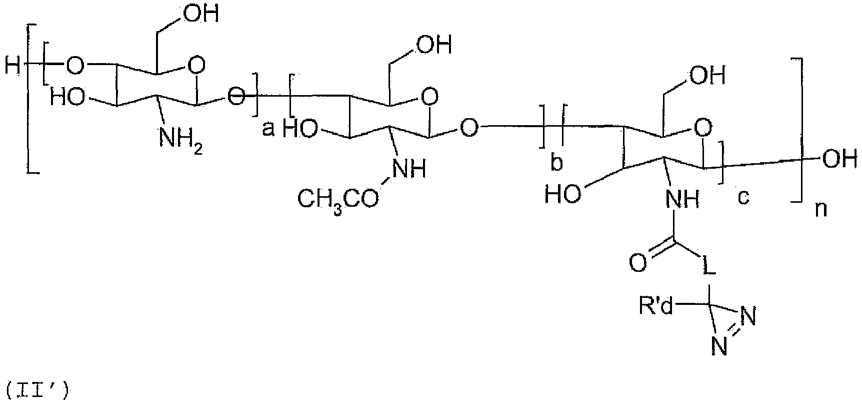 wherein
b ranges from 0 to 0.5, preferably from 0.05 to 0.30, and better still from 0.1 to 0.30;
c ranges from 0.001 to 0.5, preferably from 0.01 to 0.3, and better still from 0.01 to 0.2;
and a + b + c = 1.
L and R'd having the meanings according to Claim 15.

19. Polymer according to one of Claims 15 to 18, **characterized in that** the grafted chitosan polymer (II) is of formula (IIa): 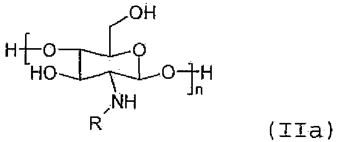 wherein R denotes H or acetyl or a group (J): 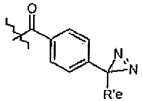 wherein R'e = CH₃ ou CF₃, the polymer comprising at least one group (J);
or of formula (IIb) 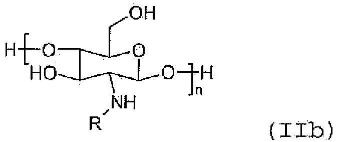 wherein R denotes H or acetyl or a group (K): 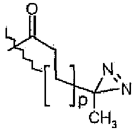 with p= 1 to 3, the polymer comprising at least one group (K).

20. Composition comprising, in a physiologically acceptable medium, a polymer (II) or (II') or (IIa) or (IIb) according to one of Claims 15 to 19.

21. Composition according to the preceding claim, **characterized in that** polymer (II) or (IIa) or (IIb) is present in a content ranging from 0.1% to 10% by weight, relative to the total weight of the composition, preferably ranging from 0.5% to 10% by weight, preferentially ranging from 1% to 8% by weight, and more preferentially ranging from 1% to 6% by weight.

22. Composition according to Claim 20 or 21, **characterized in that** it comprises a cosmetic adjuvant chosen from water, emulsifiers, preserving agents, sequestrants, fragrances, thickeners, oils, waxes and film-forming polymers.

23. Process for deodorizing the feet and/or the shoes, consisting in placing in a shoe a sole of which the surface has been treated by applying to said surface a composition comprising a grafted chitosan (I) according to one of Claims 1 to 9, then exposing the treated surface to light radiation, preferably for at least 5 seconds, according to one of Claims 1 and 12 to 14.

24. Process for deodorizing the body (or part of the body), consisting in clothing the body (or part of the body) with clothing made of textile fabric of which the surface has been treated by applying to the surface of the textile a composition comprising a grafted chitosan (I) according to one of Claims 1 to 9, and then exposing the treated surface to light radiation, preferably for at least 5 seconds, according to one of Claims 1 and 12 to 14.

25. Process according to the preceding claim, **characterized in that** the part of the body is the feet and the item of textile clothing is chosen from socks, stockings and tights.
